(19) **Europäisches Patentamt**
**European Patent Office**
**Office européen des brevets**

(11) Veröffentlichungsnummer: **0 138 756**
**B1**

(12) # EUROPÄISCHE PATENTSCHRIFT

(45) Veröffentlichungstag der Patentschrift: **04.10.89**

(51) Int. Cl.⁴: **C 07 D 413/12**, C 07 D 413/14, A 01 N 43/88

(21) Anmeldenummer: **84810396.6**

(22) Anmeldetag: **13.08.84**

(54) **Neue Oxadiazine.**

(30) Priorität: **17.08.83 CH 4487/83**
**13.04.84 CH 1861/84**
**13.07.84 CH 3424/84**

(43) Veröffentlichungstag der Anmeldung:
**24.04.85 Patentblatt 85/17**

(45) Bekanntmachung des Hinweises auf die Patenterteilung:
**04.10.89 Patentblatt 89/40**

(84) Benannte Vertragsstaaten:
**AT BE CH DE FR IT LI NL**

(56) Entgegenhaltungen:
**DE-A-2 713 712**
**DE-A-2 732 115**
**DE-A-2 905 687**

(73) Patentinhaber: **CIBA- GEIGY AG, Klybeckstrasse 141, CH- 4002 Basel (CH)**

(72) Erfinder: **Böger, Manfred, Wilhelm Glock- Strasse 14, D-7858 Weil am Rhein 5 (DE)**
Erfinder: **Drabek, Jozef, Dr., Benkenstrasse 12, CH-4104 Oberwil (CH)**

**EP 0 138 756 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die vorliegende Erfindung betrifft neue 1,3,5-Oxadiazin-2,4-dione, ihre Herstellung, ihre Verwendung in der Schädlingsbekämpfung sowie Schädlingsbekämpfungsmittel, welche diese Oxadiazine enthalten.

Die erfindungsgemässen Oxadiazine entsprechen der Formel I

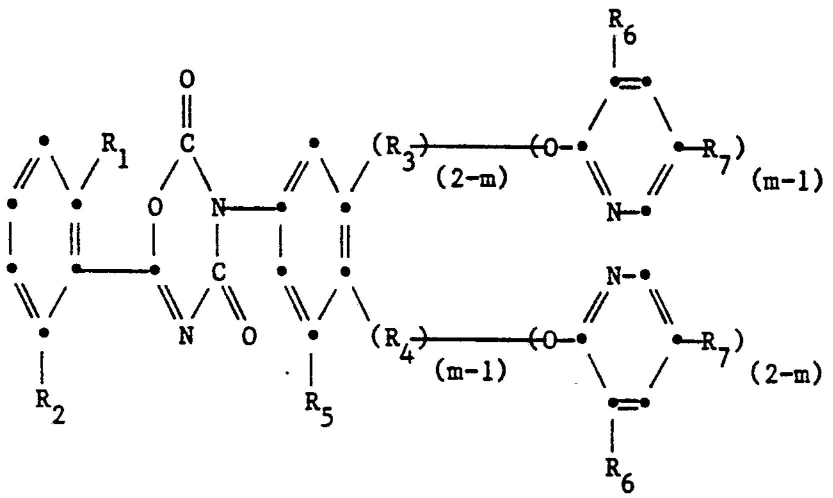

(I),

worin

$R_1$ Halogen, $C_1$-$C_4$-Alkyl, $C_1$-$C_4$-Alkoxy oder $C_1$-$C_4$-Alkylthio,
$R_2$ Wasserstoff, Halogen, $C_1$-$C_4$-Alkyl, $C_1$-$C_4$-Alkoxy oder $C_1$-$C_4$-Alkylthio,
$R_3$ und $R_4$ je Wasserstoff, Halogen oder $C_1$-$C_4$-Alkyl,
$R_5$ Wasserstoff, Halogen, $C_1$-$C_4$-Alkyl oder $-COOR_8$,
$R_6$ Wasserstoff oder Halogen,
$R_7$ ein- oder mehrfach halogeniertes $C_1$-$C_4$-Alkyl,
$R_8$ Wasserstoff oder $C_1$-$C_4$-Alkyl und
m die Zahlen 1 oder 2
bedeuten.

Bei den als Substituenten in Betracht kommenden Halogenen handelt es sich sowohl um Fluor und Chlor als auch um Brom und Jod, wobei Fluor und Chlor bevorzugt sind.

Die als Substituenten in Betracht kommenden $C_1$-$C_4$-Alkyle, $C_1$-$C_4$-Alkoxy- und $C_1$-$C_4$-Alkylthiogruppen können geradkettig oder verzweigt sein. Als Beispiele solcher niederer Alkyle seien Methyl, Methoxy, Methylthio und Äthyl, Äthoxy, Äthylthio, sowie Propyl, Propoxy, Propylthio und Butyl, Butoxy, Butylthio und ihre Isomeren erwähnt, wobei Methyl, Methoxy, Methylthio und Äthyl, Ähoxy, Ähylthio bevorzugt sind.

Die für Halogen und die $C_1$-$C_4$-Alkyle gegebenen Definitionen gelten auch für die ein- oder mehrfach halogenierten $C_1$-$C_4$-Alkyle. Beispiele solcher Halogenalkyle sind u.a. das ein- bis dreifach durch Fluor, Chlor und/oder Brom substituierte Methyl oder das ein- bis fünffach durch Fluor, Chlor und/oder Brom substituierte Äthyl.

Vornehmlich seien solche Verbindungen der Formel I genannt, in denen

$R_1$ Halogen oder $C_1$-$C_4$-Alkyl,
$R_2$ Wasserstoff oder Halogen,
$R_3$ und $R_4$ je Wasserstoff, Halogen oder $C_1$-$C_4$-Alkyl,
$R_5$ Wasserstoff, Halogen, $C_1$-$C_4$-Alkyl oder $-COOR_8$,
$R_6$ Wasserstoff oder Halogen,
$R_7$ ein- oder mehrfach halogeniertes $C_1$-$C_4$-Alkyl,
$R_8$ Wasserstoff oder $C_1$-$C_4$-Alkyl und
m die Zahlen 1 oder 2
bedeuten.

Unter diesen Verbindungen der Formel I stehen diejenigen im Vordergrund, in denen

$R_1$ Halogen oder $C_1$-$C_4$-Alkyl,
$R_2$ Wasserstoff oder Halogen,

$R_3$, $R_4$ und $R_5$ je Wasserstoff, Halogen oder $C_1$-$C_4$-Alkyl,
$R_6$ Wasserstoff oder Halogen,
$R_7$ ein- oder mehrfach halogeniertes $C_1$-$C_4$-Alkyl und
m die Zahlen 1 oder 2
bedeuten.

Dabei sind diejenigen Verbindungen der Formel I bevorzugt, in denen

$R_1$ Halogen,
$R_2$, $R_3$ und $R_5$ Wasserstoff oder Halogen,
$R_6$ Wasserstoff oder Chlor,
$R_7$ ein- oder mehrfach durch Fluor und/oder Chlor substituiertes $C_1$-$C_2$-Alkyl und
m die Zahlen 1 oder 2
bedeuten.

Besondere Erwähnung verdienen dabei diejenigen Verbindungen der Formel I, in denen

$R_1$ Chlor oder Fluor,
$R_2$ Wasserstoff oder Fluor,
$R_3$, $R_5$ und $R_6$ Wasserstoff oder Chlor,
$R_7$ $-CF_3$ oder $-CF_2CFCl_2$ und
m die Zahl 1
bedeuten.

Beispiele von Verbindungen der Formel I sind u.a.

| $R_1$ | $R_2$ | $R_3$ | $R_4$ | $R_5$ | $R_6$ | $R_7$ | m |
|---|---|---|---|---|---|---|---|
| F | H | H | - | H | H | $CF_3$ | 1 |
| F | F | F | - | F | F | $CF_3$ | 1 |
| F | Cl | Cl | - | Cl | Cl | $CF_3$ | 1 |
| F | Br | - | Br | H | Br | $CBr_3$ | 2 |
| F | F | H | - | H | Cl | $CF_2CCl_3$ | 1 |
| F | F | Cl | - | Cl | H | $CF_3$ | 1 |
| F | F | - | $n-C_4H_9$ | H | H | $CF_2$ | 2 |
| Cl | H | $n-C_4H_9$ | - | H | H | $CF_3$ | 1 |
| Cl | H | J | - | $C_2H_5$ | Br | $CF_2CF_3$ | 1 |
| Cl | F | Br | - | Br | H | $CF_2CF_3$ | 1 |
| Cl | Cl | Cl | - | Cl | Cl | $CCl_3$ | 1 |
| Cl | H | H | - | H | Cl | $CF_2CF_2Cl$ | 1 |
| Cl | Cl | H | - | H | H | $CF_3$ | 1 |
| Cl | H | Cl | - | Cl | H | $CF_3$ | 1 |
| Cl | Cl | - | $i-C_3H_7$ | H | Br | $CF_3$ | 2 |
| Cl | H | $C_2H_5$ | - | H | Cl | $CH_2CF_3$ | 1 |
| Br | Br | F | - | F | H | $CF_3$ | 1 |
| Br | J | - | H | H | Cl | $CF_3$ | 2 |
| Br | H | - | F | $n-C_3H_7$ | H | $CF_3$ | 2 |
| Br | F | $C_2H_5$ | - | Br | Cl | $CF_3$ | 1 |
| J | H | Br | - | H | H | $CHF_2$ | 1 |
| $CH_3$ | H | - | $CH_3$ | J | Cl | $CF_3$ | 2 |
| $CH_3$ | F | F | - | H | H | $CF_3$ | 1 |
| $CH_3$ | H | Cl | - | Cl | Cl | $CF_3$ | 1 |
| $t-C_4H_9$ | H | $n-C_3H_7$ | - | H | F | $CF_3$ | 1 |
| Cl | H | H | - | $COOC_2H_5$ | Cl | $CF_3$ | 1 |
| F | F | H | - | $COOCH_3$ | Cl | $CF_2CFCl_2$ | 1 |
| F | F | Cl | - | $COOCH_3$ | Cl | $CF_3$ | 1 |
| Cl | H | H | - | $COOC_4H_9$-n | Cl | $CF_3$ | 1 |
| F | F | Cl | - | $COOCH_3$ | Cl | $CF_2CFCl_2$ | 1 |
| Cl | H | Br | - | $COOCH_3$ | Cl | $CF_3$ | 1 |
| $CH_3$ | Cl | H | - | $COOCH_3$ | Cl | $CF_3$ | 1 |
| F | F | H | - | COOH | Cl | $CF_3$ | 1 |
| F | F | - | H | $COOCH_3$ | Cl | $CF_3$ | 2 |
| $CH_3O$ | H | - | H | $CH_3$ | Cl | $CF_3$ | 2 |
| $CH_3O$ | F | $n-C_4H_9$ | - | F | F | $CF_3$ | 1 |

| | | | | | | | |
|---|---|---|---|---|---|---|---|
| n-$C_4H_9O$ | Cl | F | - | H | H | $CF_2CFCl_2$ | 1 |
| $CH_3O$ | $CH_3O$ | H | - | $COOCH_3$ | Cl | $CF_3$ | 1 |
| $C_3H_7O$ | $C_3H_7O$ | Cl | - | Cl | Cl | $CF_3$ | 1 |
| i-$C_3H_7O$ | H | $CH_3$ | - | H | Cl | $CF_3$ | 1 |
| $CH_3S$ | $CH_3S$ | - | H | $COOC_2H_5$ | F | $CF_3$ | 2 |
| $C_2H_5S$ | H | Cl | - | Cl | Cl | $CF_3$ | 1 |
| $CH_3S$ | $CH_3$ | H | - | H | Cl | $CF_3$ | 1 |

Die erfindungsgemässen Verbindungen können nach an sich bekannten Verfahren hergestellt werden. Solche Verfahren sind u.a. in den DE-OS-2 732 115 und 2 905 687 beschrieben. So können die Verbindungen der Formel I z. B. erhalten werden, indem man ein Benzoylisocyanat der Formel II

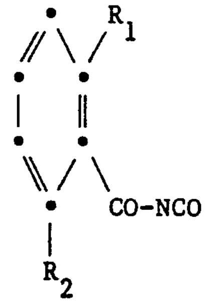

(II)

mit einem Isocyanat der Formel III

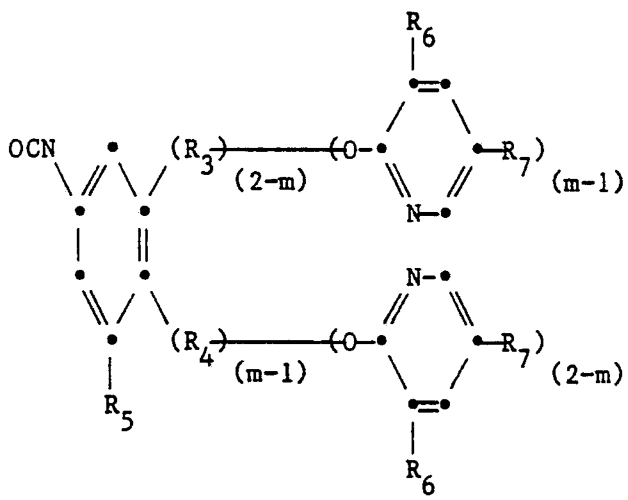

(III)

umsetzt. Zur Durchführung des Verfahrens erhitzt man die beiden Reaktionskomponenten gegebenenfalls in Gegenwart eines Lösungs- oder Verdünnungsmittels während 30 Minuten bis 30 Stunden auf eine Temperatur im Bereich von 50 bis 150°C, vorzugsweise während 5 bis 15 Stunden auf eine Temperatur im Bereich von 80 bis 120°C. Als Lösungs- oder Verdünnungsmittel eignen sich vor allem polare aprotische Lösungsmittel wie z. B. Dimethylsulfoxid, Dimethylformamid oder N,N-Dimethylacetamid.

Die erfindungsgemässen Verbindungen können aber auch nach anderen Methoden hergestellt werden, z. B. indem man

a) ein Halogencarbonylbenzamid der Formel IV

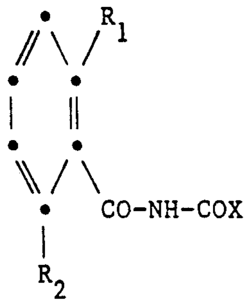

(IV)

mit einem Isocyanat der Formel III, oder

b) ein Benzoylisocyanat der Formel II mit einem Carbaminsäureester der Formel V

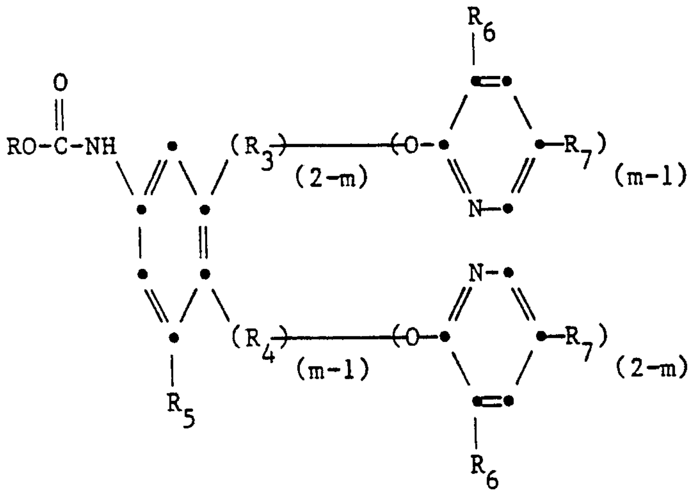

(V)

kondensiert. In den Formeln II bis V haben die Reste $R_1$ bis $R_7$ und m die für Formel I angegebene Bedeutung, während X für Halogen, vorzugsweise Chlor, und R für einen Alkylrest, vorzugsweise einen niederen Alkylrest mit 1 bis 4 Kohlenstoffatomen, steht.

Die Verbindungen der Formeln II bis V sind bekannt oder können nach bekannten Methoden hergestellt werden. So können die Benzoylisocyanate der Formel II durch Umsetzung der entsprechend substituierten Benzamide mit Oxalylchlorid hergestellt und die Isocyanate der Formel III aus den entsprechend substituierten Pyridyloxyanilinen durch Umsetzung mit Phosgen gewonnen werden.

Die erfindungsgemässen Verbindungen sind bei günstiger Warmblüter- und Pflanzenverträglichkeit wertvolle Wirkstoffe in der Schädlingsbekämpfung. So eignen sich die Verbindungen der Formel I z. B. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und Arachniden der Ordnung Acarina, wie z. B. Milben und Zecken. Dabei kann jedes Entwicklungsstadium der Schädlinge bekämpft werden, d.h. sowohl die Adulten, Puppen und Nymphen, als auch insbesondere die Larven und Eier. So können vor allem Larven und Eier von phytopathogenen Schadinsekten und -milben in Zier- und Nutzpflanzungen, wie z. B. in Obst- und Gemüsepflanzungen, und insbesondere in Baumwollpflanzungen, wirkungsvoll bekämpft werden. Werden Verbindungen der Formel I von Imagines aufgenommen, so kann sich ihre Wirkung in unmittelbarer Abtötung der Schädlinge zeigen oder aber in verminderter Eiablage und/oder Schlupfrate. Die letztere Erscheinung kann insbesondere bei Coleopteren beobachtet werden. Bei der Bekämpfung von tierparasitären Schädlingen, insbesondere an Haus- und Nutztieren, kommen vor allem Ektoparasiten, wie z. B. Milben und Zecken und Dipteren, wie z. B. Lucilia sericata in Betracht.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als solche Zusätze kommen z. B. organische Phosphorverbindungen, Nitrophenole und Derivate, Formamidine, Harnstoffe, Carbamate, Pyrethroide, chlorierte Kohlenwasserstoffe und Bacillus thuringiensis-Präparate in Betracht.

Mit besonderem Vorteil kann man die Verbindungen der Formel I auch mit Substa... sind ... einen pestizid verstärkenden Effekt ausüben. Beispiele solcher Verbindungen sind ... Propinyläther, Propinyloxime, Propinylcarbamate und Propinylphosphonate, 2-(3,4-Methy... 3,6,9-trioxaundecan oder S,S,S-Tributylphosphorotrithioate.

Die Verbindungen der Fomel I werden in unveränderter Form oder vorzugsweise zusammen mit ... Formulierungstechnik üblichen Hilfsmitteln eingesetzt und werden daher z. B. zu Emulsionskonzentraten, ... versprühbaren oder verdünnbaren Lösungen, verdünnten Emulsionen, Spritzpulvern, löslichen Pulver... Stäubemitteln, Granulaten oder auch Verkapselungen in z. B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierungen, d.h. die einen Wirkstoff der Formel I bzw. Kombinationen dieser Wirkstoffe mit andern Insektiziden oder Akariziden, und gegebenenfalls einen festen oder flüssigen Zusatzstoff enthaltenden Mittel, Zubereitungen oder Zusammensetzungen, werden in bekannter Weise hergestellt, z. B. durch inniges Vermischen und/oder Vermahlen der Wirkstoffe mit Streckmitteln, wie z. B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$ wie z. B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Äther und Ester, wie Äthanol, Äthylenglykol, Äthylenglykolmonomethyl oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z. B. Bimsstein, Ziegelbruch, Sepiolit oder Bentonit, als nicht sorptive Trägermaterialien z. B. Calcit oder Sand, in Frage. Darüberhinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombination dieser Wirkstoffe mit anderen Insektiziden oder Akariziden nichtionogene, kation- und/oder anionaktive Tenside mit guten Emulgier-, Dispergier-und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z. B. die Na- oder K-Salze der Öl- oder Stearinsäure, oder von natürlichen Fettsäuregemischen, die z. B. aus Kokosnuss- oder Tallöl gewonnen werden können. Ferner sind die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch Alkylteil von Acylresten einschliesst, z. B. das Na- oder Ca-Salz der Ligninsulfonsäure, des Dodecyl-
...lsäureesters oder eines aus natürlichen Fettsäuren hergestellten Fettalkoholsulfatgemisches. Hierher
...h die Salze der Schwefelsäureester und Sulfonsäuren von Fettalkohol-Äthylenoxid-Addukten. Die
...imidazolderivate enthalten vorzugsweise 2 Sulfonsäuregruppen und einen Fettsäurerest mit
...n. Arylalkylsulfonate sind z. B. die Na-, Ca- oder Triäthanolaminsalze der Dodecylben-
...ylnaphthalinsulfonsäure oder eines Naphthalinsulfonsäure-Formaldehydkondensa-
...en auch entsprechende Phosphate, wie z. B. Salze des Phosphorsäureesters eines
...-Adduktes in Frage.

... in erster Linie Polyglykolätherderivate von aliphatischen oder cycloali-
...ungesättigten Fettsäuren und Alkylphenolen in Frage, die 3 bis 30
...ffatome im (aliphatischen) Kohlenwasserstoffrest und 6 bis 18
...nthalten können. Weiterhin geeignete nichtionische Tenside
...ergruppen und 10 bis 100 Propylenglyk...äthergruppen
...lykol, Äthylendiaminopolypropylengl... Alkyl-
...ylkette. Die genannten Verbind...
...iten.
...oxyäthanole, Ricinus...
...l. Polyäthyleng...
... Polyoxyäthy...

Substituenten mindestens einen Alkylrest mit 8 bis 22 C-Atomen enthalten und als weitere Substituenten niedrige, gegebenenfalls halogenierte Alkyl-, Benzyl -oder niedrige Hydroxyalkylreste aufweisen. Die Salze liegen vorzugsweis als Halogenide, Methylsulfate oder Äthylsulfate vor, z. B. das Stearyltrimethylammoniumchlorid oder das Benzyl-di-(2-chloräthyl)-äthylammoniumbromid.

Die in der Formulierungstechnik gebräuchlichen Tenside sind u.a. in den folgenden Publikationen beschrieben:

"Mc Cutcheon's Detergents and Emulsifiers Annual" MC Publishing Corp., Ridgewood, New Jersey, 1981; Dr. Helmut Stache "Tensid-Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit anderen Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze, wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe der Formel I oder Kombinationen dieser Wirkstoffe mit andern Insektiziden oder Akariziden (% = Gewichtsprozent):

| 1. **Emulsionskonzentrate** | a) | b) | c) |
|---|---|---|---|
| Wirkstoff oder Wirkstoffkombination | 25 % | 40 % | 50 % |
| Ca-Dodecylbenzolsulfonat | 5 % | 8 % | 6 % |
| Ricinusölpolyäthylenglykoläther (36 Mol ÄO) | 5 % | - | - |
| Tributylphenolpolyäthylenglykoläther (30 Mol ÄO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. **Lösungen** | a) | b) | c) | d) |
|---|---|---|---|---|
| Wirkstoff oder Wirkstoffkombination | 80 % | 10 % | 5 % | 95 % |
| Äthylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160 - 190° C) | - | - | 94 % | - |

Die Lösungen sind zur Anwendung in Form kleinster Tropfen geeignet.

| 3. **Granulate** | a) | b) |
|---|---|---|
| Wirkstoff oder Wirkstoffkombination | 5 % | 10 % |
| Kaolin | 94 % | - |
| Hochdisperse Kieselsäure | 1 % | - |
| Attapulgit | - | 90 % |

Der Wirkstoff oder die Wirkstoffkombination wird in Methylenchlorid gelöst, auf den Träger aufgesprüht und das Lösungsmittel anschliessend im Vakuum abgedampft.

| 4. **Stäubemittel** | a) | b) |
|---|---|---|
| Wirkstoff oder Wirkstoffkombination | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff oder der Wirkstoffkombination erhält man gebrauchsfertige Stäubemittel.

Formulierungen für feste Wirkstoffe der Formel I oder Kombinationen dieser Wirkstoffe mit anderen Insektiziden oder Akarizide (% = Gewichtsprozent):

5. **Spritzpulver**

| | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7 - 8 Mol ÄO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. **Emulsions-Konzentrat**

| | |
|---|---|
| Wirkstoff oder Wirkstoffkombination | 10 % |
| Octylphenolpolyäthylenglykoläther (4 - 5 Mol ÄO) | 3 % |
| Ca-Dodecylbenzolsulfonat | 3 % |
| Ricinusölpolyglykoläther (36 Mol ÄO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

7. **Stäubemittel**

| | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff oder die Wirkstoffkombination mit dem Träger vermischt und auf einer geeigneten Mühle vermahlen wird.

8. **Extruder-Granulat**

| | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zuschlagstoffen vermischt, vermahlen und mit Wasser angefeuchtet. Dieses Gemisch wird extrudiert, granuliert und anschliessend im Luftstrom getrocknet.

9. **Umhüllungs-Granulat**

| | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

10. **Suspensions-Konzentrat**

| | |
|---|---|
| Wirkstoff oder Wirkstoffkombination | 40 % |
| Äthylenglykol | 10 % |
| Nonylphenolpolyäthylenglykoläther (15 Mol ÄO) | 6 % |
| Na-Ligninsulfonat | 10 % |
| Carboxymethylcellulose | 1 % |
| 37 %-ige wässrige Formaldehyd-Lösung · | 0,2 % |
| Silikonöl in Form einer 75 %-igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

In den nachfolgenden biologischen Beispielen bedeutet eine gute Wirkung, dass der erwünschte Effekt zu mindestens 50 bis 60 % eintritt.

**Beispiel 1:**

a) 4-[3-Chlor-5-(1'-difluor-2'-dichlorfluoräthyl)-2-pyridyloxy]-anilin

Es werden 1,3 g gepulvertes Kaliumhydroxid mit 10 ml Dimethylsulfoxid vermischt. Nach Zugabe von 2,15 g 4-Nitrophenol wird das Gemisch 1 Stunde bei 100° C gerührt. Das Gemisch wird dann auf 50° C abgekühlt. Danach werden 5,1 g 2,3-Dichlor-5-(1'-difluor-2'-dichlor-fluoräthyl)-pyridin, gelöst in 5 ml Dimethylsulfoxid, hinzugetropft, und unter einer Stickstoffatmosphäre wird 5 Stunden bei 120° C gerührt. Das Reaktionsgemisch wird auf Eiswasser gegossen, mit Toluol extrahiert, die Toluolphase abgetrennt, mit Wasser gewaschen, getrocknet und eingedampft. Man erhält das 4-[3-Chlor-5-(1'-difluor-2'-dichlorfluoräthyl)-2-pyridyloxy]-nitrobenzol als weisses Pulver vom Schmelzpunkt 100 - 102° C.

5,3 g 4-[3-Chlor-5-(1'-difluor-2'-dichlorfluoräthyl)-2-pyridyloxy]-nitrobenzol werden mit 15 ml konz. Chlorwasserstoffsäure vermischt. Bei 70 - 75° C werden 13,1 g Zinndichlorid ($SnCl_2 \cdot 2H_2O$) in 20 ml konz. Chlorwasserstoffsäure tropfenweise zugegeben. Das Gemisch wird bei ca. 100° C gerührt. Danach wird das Reaktionsgemisch auf Eis gegossen, mit Natronlauge (50 Gew.-% NaOH) alkalisch gestellt, mit Dichlormethan extrahiert, der erhaltene Extrakt neutral gewaschen, getrocknet, stark eingeengt und über Kieselgel filtriert. Das erhaltene Filtrat wird eingedampft, und man erhält das 4-[3-Chlor-5-(1'-difluor-2'-dichlorfluoräthyl)-2-pyridyloxy]-anilin als weisse Kristalle vom Smp. 87 - 88° C.

b) 42,3 g 2,3-Dichlor-5-(1'-difluor-2'-dichlorfluoräthyl)-pyridin werden in 30 ml Dimethylsulfoxid gelöst. Im Verlaufe von 30 Minuten wird diese Lösung bei 90° C und unter einer Stickstoffatmosphäre tropfenweise in eine Mischung von 16,2 g 4-Aminophenol, 33,2 g Kaliumcarbonat und 140 ml Dimethylsulfoxid eingerührt. Das Reaktionsgemisch wird 2 Stunden lang unter diesen Bedingungen weitergerührt und dann am Rotationsverdampfer unter Vakuum vom Dimethylsulfoxid befreit. Der Rückstand wird mit Dichlormethan und Wasser versetzt und die organische Phase mehrmals mit Wasser gewaschen, getrocknet, auf die Hälfte eingeengt und dann über Kieselgel filtriert. Das Lösungsmittel wird weitgehend aus dem Filtrat abgedampft und der Rückstand mit Hexan versetzt. Man erhält das 4-[3-Chlor-5-(1'-difluor-2'-dichlorfluoräthyl)-2-pyridyloxy]-anilin in Form weisser Kristalle, die einen Schmelzpunkt von 87 - 88° C aufweisen.

c) 4-[3-Chlor-5-(1'-difluor-2'-dichlorfluoräthyl)-2-pyridyloxy]-phenylisocyanat

1,5 g 4-[3-Chlor-5-(1'-difluor-2'-dichlorfluoräthyl)-2-pyridyloxy]-anilin werden in 30 ml Chlorbenzol gelöst und tropfenweise unter Rühren bei 22° C in eine Lösung bestehend aus 15 g 20 Gew.-% Phosgen enthaltendem Toluol, 20 ml Dioxan und 60 ml Chlorbenzol gegeben, wobei die Reaktionstemperatur auf 28° C steigt. Das Reaktionsgemisch wird dann bei Raumtemperatur eine Stunde lang und darauf 90 Minuten lang auf dem Wasserbad bei 50° C nachgerührt. Anschliessend wird am Wasserstrahlvakuum eingeengt und am Hochvakuum bei 60° C getrocknet.

Man erhält die Titelverbindung der Formel

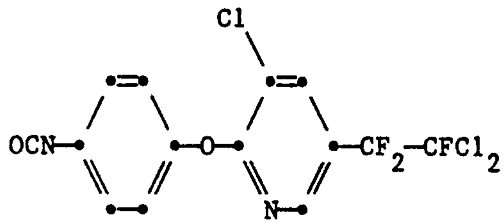

als gelbes Öl.

**Beispiel 2:**

3-[4-(3-Chlor-5-(1'-difluor-2'-dichlorfluoräthyl)-2-pyridyloxy)-phenyl]-6-(2-chlorphenyl)-3,4-dihydro-2H-1,3,5-oxadiazin-2,4-dion

Unter Feuchtigkeitsausschluss werden 8,1 g 4-[3-Chlor-5-(1'-difluor-2'-dichlorfluoräthyl)-2-pyridyloxy]-phenylisocyanat unter Rühren mit 3,7 g Chlorbenzoylisocyanat versetzt. 12 Stunden lang wird die Temperatur auf 120° C gehalten, wobei so lange gerührt wird, bis das Reaktionsgemisch zu erstarren beginnt. Wenn sich der entstandene Kristallbrei auf Raumtemperatur abgekühlt hat, wird er mit Hexan zerrieben und anschliessend abgenutscht. Der Rückstand wird aus Toluol/Chlorbenzol (10 : 1) umkristallisiert. Man erhält die Titelverbindung der Formel

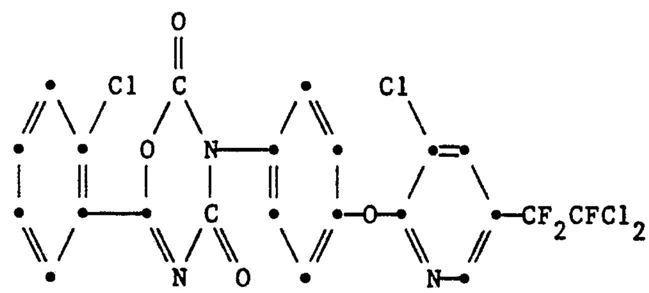

(Verb. Nr. 1)

mit einem Smp. von 198 - 199° C.

In analoger Weise werden auch die folgenden Verbindungen hergestellt:

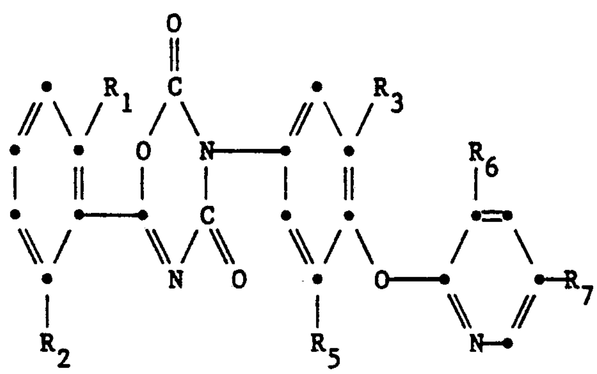

| Verb. Nr. | $R_1$ | $R_2$ | $R_3$ | $R_5$ | $R_6$ | $R_7$ | Phys. Daten |
|---|---|---|---|---|---|---|---|
| 2 | Cl | H | H | H | H | $-CF_3$ | Smp. 167 - 169° C |
| 3 | Cl | H | H | H | Cl | $-CF_3$ | Smp. 194 - 196° C |
| 4 | F | F | H | H | Cl | $-CF_2CFCl_2$ | Smp. 194 - 195° C |
| 5 | Cl | H | Cl | Cl | Cl | $-CF_3$ | Smp. 176 - 178° C |
| 6 | F | F | Cl | Cl | Cl | $-CF_3$ | Smp. 188 - 190° C |
| 7 | F | F | H | H | Cl | $-CF_3$ | Smp. 182 - 183° C |
| 8 | F | F | Cl | Cl | H | $-CF_3$ | Smp. 171 - 173° C |

| | | | | | | | |
|---|---|---|---|---|---|---|---|
| 9 | F | F | $CH_3$ | H | Cl | $-CF_3$ | Smp. 181 - 183°C |
| 10 | Cl | H | H | H | H | $-CF_3$ | Smp. 164 - 166°C |
| 11 | Cl | H | Cl | Cl | H | $-CF_3$ | Smp. 125 - 128°C |
| 12 | Br | H | Cl | Cl | Cl | $-CF_3$ | Smp. 181 - 183°C |
| 13 | $CH_3$ | H | Cl | Cl | Cl | $-CF_3$ | Smp. 181 - 182°C |
| 14 | F | H | H | $COOCH_3$ | Cl | $-CF_3$ | Smp. 185 - 187°C |
| 15 | Cl | H | H | $COOCH_3$ | Cl | $-CF_3$ | Smp. 181 - 182°C |
| 16 | F | F | H | $COOCH_3$ | Cl | $-CF_3$ | Smp. 191 - 193°C |
| 17 | $CH_3O$ | H | H | $COOCH_3$ | Cl | $-CF_3$ | Smp. 175°C |
| 18 | $CH_3O$ | H | Cl | Cl | Cl | $-CF_3$ | Smp. 188 - 190°C |
| 19 | $CH_3O$ | H | $CH_3$ | H | Cl | $-CF_3$ | Smp. 202 - 204°C |
| 20 | $CH_3S$ | F | Cl | Cl | Cl | $-CF_3$ | Smp. 155 - 175°C |
| 21 | 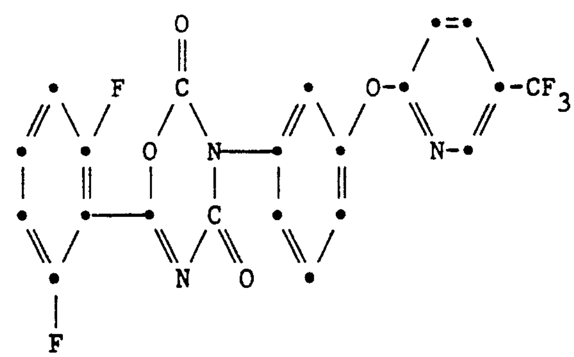 | | | | | | Smp. 174 - 178°C |

**Beispiel 3:**

Wirkung gegen Musca domestica

Je 50 g frisch zubereitetes CSMA-Nährsubstrat für Maden werden in Becher eingewogen. Von einer 1 gew.-%-igen acetonischen Lösung des betreffenden Wirkstoffes wird eine bestimmte Menge auf das in den Bechern befindliche Nährsubstrat pipettiert. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen gemäss Beispiel 2 zeigen gute Wirkung im obigen Test.

**Beispiel 4:**

Wirkung gegen Lucilia sericata

Zu 9 ml eines Zuchtmediums wird bei 50°C 1 ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen gemäss Beispiel 2 zeigen in diesem Test gute Wirkung gegen Lucilia sericata.

**Beispiel 5:**

Wirkung gegen Aedes aegypti

Auf die Oberfläche von 150 ml Wasser, das sich in einem Behälter befindet, wird soviel einer 0,1 %-igen acetonischen Lösung des Wirkstoffes pipettiert, dass eine Konzentration von 12,5 ppm erhalten wird. Nach Verdunsten des Acetons wird der Behälter mit 30 - 40 2-tägigen Aedes-Larven beschickt. Nach 2 und 7 Tagen wird die Mortalität geprüft.

Verbindungen gemäss Beispiel 2 zeigen gute Wirkung in diesem Test.

**Beispiel 6:**

Insektizide Frassgift-Wirkung

Baumwollpflanzen (ca. 20 cm hoch) werden mit einer wässrigen Wirkstoffemulsion (erhalten aus einem 10 %-gen emulgierbaren Konzentrat) besprüht, wobei die Wirkstoffemulsion 100 ppm der zu prüfenden Verbindung enthält.

Nach dem Antrocknen des Belages werden die Baumwollpflanzen je mit Spodoptera littoralis- und Heliothis virescens-Larven im dritten larvalen Stadium besetzt. Der Versuch wird bei 24°C und 60 % relativer Luftfeuchtigkeit durchgeführt. In Abständen von jeweils 24 Stunden werden Mortalität sowie Entwicklungs- und Häutungsstörungen der angesetzten Larven bestimmt.

Verbindungen gemäss Beispiel 2 zeigen gute Wirkung in diesem Test.

**Beispiel 7:**

Wirkung auf Spodoptera littoralis und Heliothis virescens (Larven und Eier)

Es werden drei in Töpfen gezogene Baumwollpflanzen von ca. 15 - 20 cm Höhe mit einer sprühfähigen flüssigen Zubereitung des zu prüfenden Wirkstoffes behandelt. Nach Antrocknen des Sprühbelages werden die eingetopften Pflanzen in ein Blechgefäss von etwa 20 Litern Inhalt gestellt, das mit einer Glasplatte abgedeckt wird. Die Feuchtigkeit im Inneren des abgedeckten Gefässes wird so reguliert, dass sich kein Kondenswasser bildet. Direktes, auf die Pflanzen fallendes Licht wird vermieden. Dann werden die drei Pflanzen infestiert, und zwar insgesamt mit:

a) 50 Larven von Spodoptera littoralis oder Heliothis virescens des ersten larvalen Stadiums;
b) 20 Larven von Spodoptera littoralis oder Heliothis virescens des dritten larvalen Stadiums;
c) zwei Eispiegeln von Spodoptera littoralis oder Heliothis virescens (dazu werden je 2 Blätter einer Pflanze in einem beidseitig mit Gaze verschlossenen Plexiglaszylinder eingeschlossen); zwei Eispiegel von Spodoptera oder ein Teil eines Baumwollblattes mit darauf abgelegten Eiern von Heliothis werden zu den eingeschlossenen Blättern gegeben.

Nach 4 und 5 Tagen erfolgt die Auswertung gegenüber unbehandelten Kontrollen unter Berücksichtigung folgender Kriterien:

a) Anzahl der noch lebenden Larven,
b) larvale Entwicklungs- und Häutungshemmung,
c) Fraßschaden (Schabfrass und Lochfrass),
d) Schlupfrate (Anzahl der aus den Eiern geschlüpften Larven).

Verbindungen gemäss Beispiel 2 zeigen in einer Konzentration von 400 ppm gute Gesamt-Wirksamkeit in obigem Test.

**Beispiel 8:**

Ovizide Wirkung auf Spodoptera littoralis

Auf Filterpapier abgelegte Eier von Spodoptera littorlis werden aus dem Papier ausgeschnitten und in eine 0,05 gew.-%-ige Lösung des Wirkstoffes in einem Aceton-Wasser-Gemisch (1 : 1) getaucht. Die so behandelten Eiablagen werden dann aus diesem Gemisch herausgenommen und bei 28°C und 60 % relativer Feuchtigkeit in Kunststoffschalen deponiert.

Nach 5 Tagen wird die Schlupfrate, d.h. die Anzahl Larven, die sich aus den behandelten Eiern entwickelt haben, bestimmt. .

Verbindungen gemäss Beispiel 2 zeigen gute Wirkung im obigen Test.

**Beispiel 9:**

Wirkung auf Laspeyresia pomonella (Eier)

Abgelegte Eier von Laspeyresia pomonella, die nicht älter als 24 Stunden sind, werden auf Filterpapier für 1 Minute in eine acetonisch-wässrige Lösung, enthaltend 400 ppm des zu prüfenden Wirkstoffes, eingetaucht. Nach dem Antrocknen der Lösung werden die Eier in Petrischalen ausgelegt und bei einer Temperatur von 28°C belassen. Nach 6 Tagen wird der prozentuale Schlupf aus den behandelten Eiern bewertet.

Verbindungen gemäss Beispiel 2 zeigen gute Wirkung in obigem Test.

**Beispiel 10:**

Reproduktions-Beeinflussung von Anthonomus grandis

Adulte Anthonomus grandis, die nach dem Schlupf nicht älter als 24 Stunden sind, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 0,1 gew.-% des zu prüfenden Wirkstoffes, eingetaucht.

Wenn die Käfer wieder trocken sind, werden sie zur Kopulation und Eiablage in abgedeckte und Futter enthaltende Schalen eingesetzt.

Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraumes von etwa vier Wochen überprüft. Die Bonitierung erfolgt anhand der Verminderung der Anzahl abgelegter Eier und der daraus geschlüpften Larven im Vergleich zu unbehandelten Kontrollen. Verbindungen gemäss Beispiel 2 zeigen eine gute reproduktionsreduzierende Wirkung im obigen Test.

**Beispiel 11:**

Wirkung gegen Anthonomus grandis (Adulte)

Zwei eingetopfte Baumwollpflanzen im 6-Blattstadium werden mit wässrigen benetzungsfähigen Emulsions-Zubereitungen, enthaltend 100 ppm des zu prüfenden Wirkstoffes, besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Öffnungen mit Gaze abgedeckt sind, werden dann über die behandelten, mit den Testtieren besiedelten Pflanzen gestülpt, um ein Abwandern der Käfer zu verhindern. Die behandelten Pflanzen werden bei 25°C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen gemäss Beispiel 2 zeigen gute Wirkung in diesem Test.

**Patentansprüche** für die Vertragsstaaten: BE, CH, LI, DE, FR, IT, NL

1. Verbindungen der Formel I

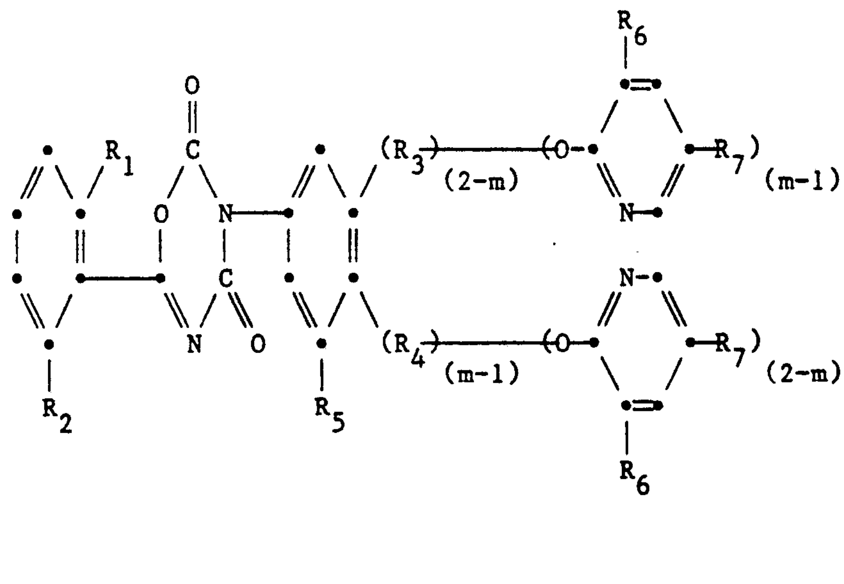

(I),

worin

$R_1$ Halogen, $C_1$-$C_4$-Alkyl, $C_1$-$C_4$-Alkoxy oder $C_1$-$C_4$-Alkylthio,

$R_2$ Wasserstoff, Halogen, $C_1$-$C_4$-Alkyl, $C_1$-$C_4$-Alkoxy oder $C_1$-$C_4$-Alkylthio,

$R_3$ und $R_4$ je Wasserstoff, Halogen oder $C_1$-$C_4$-Alkyl,
$R_5$ Wasserstoff, Halogen, $C_1$-$C_4$-Alkyl oder $-COOR_8$,
$R_6$ Wasserstoff oder Halogen,
$R_7$ ein- oder mehrfach halogeniertes $C_1$-$C_4$-Alkyl,
$R_8$ Wasserstoff oder $C_1$-$C_4$-Alkyl und
m die Zahlen 1 oder 2
bedeuten.

2. Verbindungen der Formel I gemäss Anspruch 1, worin
$R_1$ Halogen oder $C_1$-$C_4$-Alkyl,
$R_2$ Wasserstoff oder Halogen,
$R_3$ und $R_4$ je Wasserstoff, Halogen oder $C_1$-$C_4$-Alkyl
$R_5$ Wasserstoff, Halogen, $C_1$-$C_4$-Alkyl oder $-COOR_8$,
$R_6$ Wasserstoff oder Halogen,
$R_7$ ein- oder mehrfach halogeniertes $C_1$-$C_4$-Alkyl,
$R_8$ Wasserstoff oder $C_1$-$C_4$-Alkyl und
m die Zahlen 1 oder 2
bedeuten.

3. Verbindungen der Formel I gemäss Anspruch 2, worin
$R_1$ Halogen oder $C_1$-$C_4$-Alkyl,
$R_2$ Wasserstoff oder Halogen,
$R_3$, $R_4$ und $R_5$ je Wasserstoff, Halogen oder $C_1$-$C_4$-Alkyl,
$R_6$ Wasserstoff oder Halogen,
$R_7$ ein- oder mehrfach halogeniertes $C_1$-$C_4$-Alkyl und
m die Zahlen 1 oder 2
bedeuten.

4. Verbindungen der Formel I gemäss Anspruch 3, worin $R_1$ Halogen, $R_2$, $R_3$, $R_4$ und $R_5$ Wasserstoff oder Halogen, $R_6$ Wasserstoff oder Chlor, $R_7$ ein- oder mehrfach durch Fluor und/oder Chlor substituiertes $C_1$-$C_2$-Alkyl und m die Zahlen 1 oder 2 bedeuten.

5. Verbindungen der Formel I gemäss Anspruch 4, worin $R_1$ Chlor oder Fluor, $R_2$ Wasserstoff oder Fluor, $R_3$, $R_5$ und $R_6$ Wasserstoff oder Chlor, $R_7$ $-CF_3$ oder $-CF_2CFCl_2$ und m die Zahl 1 bedeuten.

6. Verbindung gemäss Anspruch 5 der Formel

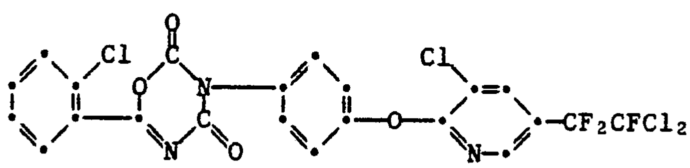

7. Verbindung gemäss Anspruch 5 der Formel

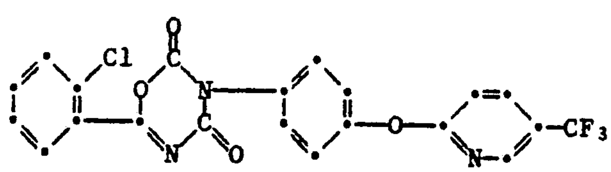

8. Verbindung gemäss Anspruch 5 der Formel

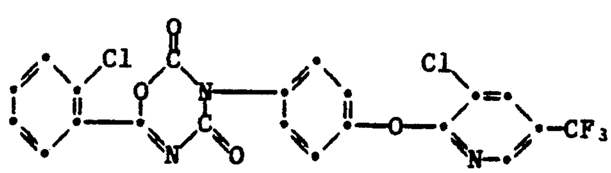

9. Verbindung gemäss Anspruch 5 der Formel

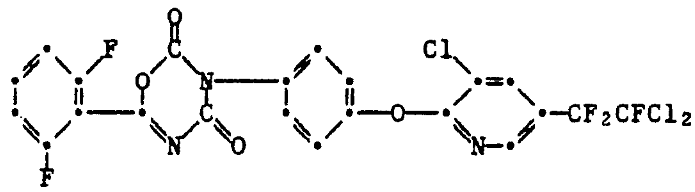

10. Verbindung gemäss Anspruch 5 der Formel

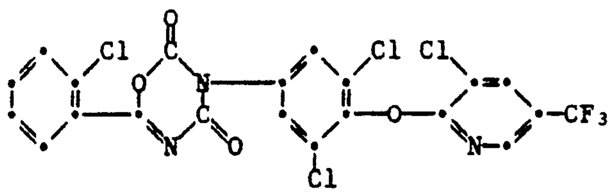

11. Verbindung gemäss Anspruch 5 der Formel

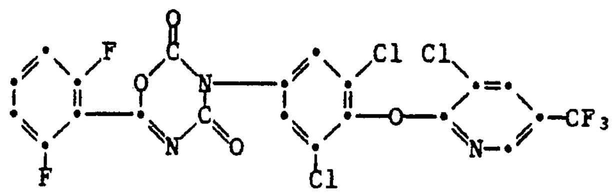

12. Verbindung gemäss Anspruch 5 der Formel

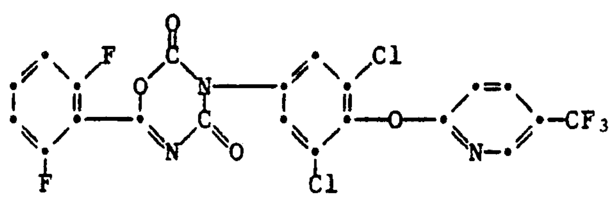

13. Verfahren zur Herstellung einer Verbindung der Formel I

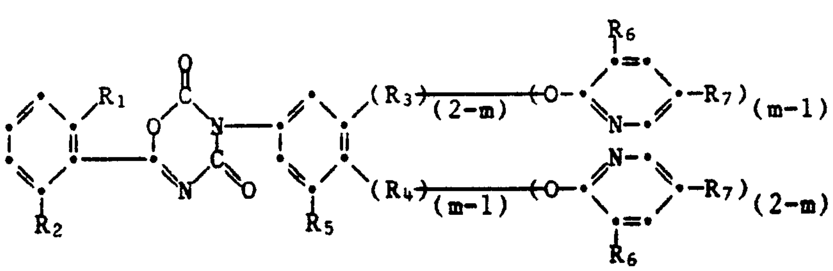

(I),

worin
$R_1$ Halogen, $C_1$-$C_4$-Alkyl, $C_1$-$C_4$-Alkoxy oder $C_1$-$C_4$-Alkylthio,
$R_2$ Wasserstoff, Halogen, $C_1$-$C_4$-Alkyl, $C_1$-$C_4$-Alkoxy oder $C_1$-$C_4$-Alkylthio,
$R_3$ und $R_4$ je Wasserstoff, Halogen oder $C_1$-$C_4$-Alkyl,
$R_5$ Wasserstoff, Halogen, $C_1$-$C_4$-Alkyl oder -$COOR_8$,
$R_6$ Wasserstoff oder Halogen,
$R_7$ ein- oder mehrfach halogeniertes $C_1$-$C_4$-Alkyl,
$R_8$ Wasserstoff oder $C_1$-$C_4$-Alkyl und
m die Zahlen 1 oder 2
bedeuten, dadurch gekennzeichnet, dass man ein Benzoylisocyanat der Formel II

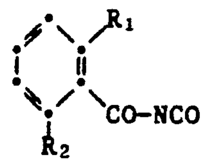

(II)

mit einem Isocyanat der Formel III

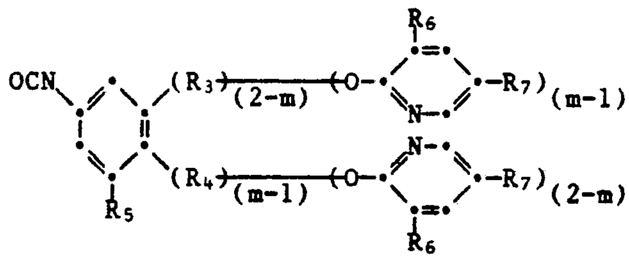

(III)

in bekannter Weise umsetzt, wobei in den Formeln II und III die Symbole $R_1$, $R_2$, $R_3$, $R_4$, $R_5$, $R_6$, $R_7$ und m die für Formel I angegebene Bedeutung haben.

14. Verfahren gemäss Anspruch 13 zur Herstellung einer Verbindung der Formel I, dadurch gekennzeichnet, dass in der Formel I
$R_1$ Halogen oder $C_1$-$C_4$-Alkyl,
$R_2$ Wasserstoff oder Halogen,
$R_3$ und $R_4$ je Wasserstoff, Halogen oder $C_1$-$C_4$-Alkyl,
$R_5$ Wasserstoff, Halogen, $C_1$-$C_4$-Alkyl oder -$COOR_8$,
$R_6$ Wasserstoff oder Halogen,
$R_7$ ein- oder mehrfach halogeniertes $C_1$-$C_4$-Alkyl,
$R_8$ Wasserstoff oder $C_1$-$C_4$-Alkyl und
m die Zahlen 1 oder 2
bedeuten.

15. Verfahren gemäss Anspruch 14 zur Herstellung einer Verbindung der Formel I, dadurch gekennzeichnet, dass in der Formel I
$R_1$ Halogen oder $C_1$-$C_4$-Alkyl,
$R_2$ Wasserstoff oder Halogen,
$R_3$, $R_4$ und $R_5$ je Wasserstoff, Halogen oder $C_1$-$C_4$-Alkyl,
$R_6$ Wasserstoff oder Halogen,
$R_7$ ein- oder mehrfach halogeniertes $C_1$-$C_4$-Alkyl und
m die Zahlen 1 oder 2
bedeuten.

16. Schädlingsbekämpfungsmittel, welches als aktive Komponente eine Verbindung der Formel I

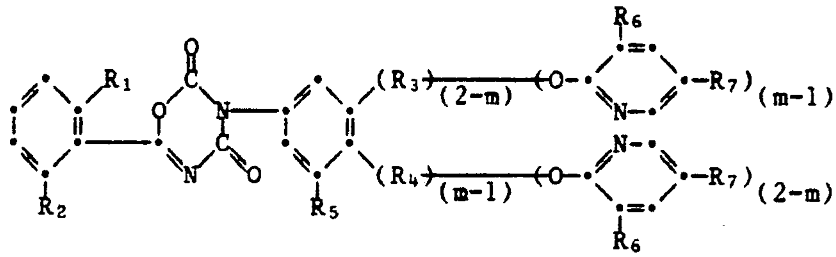

(I),

enthält, worin
$R_1$ Halogen, $C_1$-$C_4$-Alkyl, $C_1$-$C_4$-Alkoxy oder $C_1$-$C_4$-Alkylthio,
$R_2$ Wasserstoff, Halogen, $C_1$-$C_4$-Alkyl, $C_1$-$C_4$-Alkoxy oder $C_1$-$C_4$-Alkylthio,
$R_3$ und $R_4$ je Wasserstoff, Halogen oder $C_1$-$C_4$-Alkyl,
$R_5$ Wasserstoff, Halogen, $C_1$-$C_4$-Alkyl oder -$COOR_8$,
$R_6$ Wasserstoff oder Halogen,
$R_7$ ein- oder mehrfach halogeniertes $C_1$-$C_4$-Alkyl,
$R_8$ Wasserstoff oder $C_1$-$C_4$-Alkyl und
m die Zahlen 1 oder 2
bedeuten, zusammen mit geeigneten Trägern und/oder Zuschlagstoffen.

17. Schädlingsbekämpfungsmittel gemäss Anspruch 16, welches als aktive Komponente eine Verbindung der Formel I enthält, worin
$R_1$ Halogen oder $C_1$-$C_4$-Alkyl,
$R_2$ Wasserstoff oder Halogen,
$R_3$ und $R_4$ je Wasserstoff, Halogen oder $C_1$-$C_4$-Alkyl,
$R_5$ Wasserstoff, Halogen, $C_1$-$C_4$-Alkyl oder -$COOR_8$,
$R_6$ Wasserstoff oder Halogen,
$R_7$ ein- oder mehrfach halogeniertes $C_1$-$C_4$-Alkyl,
$R_8$ Wasserstoff oder $C_1$-$C_4$-Alkyl und
m die Zahlen 1 oder 2
bedeuten.

18. Schädlingsbekämpfungsmittel gemäss Anspruch 17, welches als aktive Komponente eine Verhindung der Formel I enthält, worin
$R_1$ Halogen oder $C_1$-$C_4$-Alkyl,
$R_2$ Wasserstoff oder Halogen,
$R_3$, $R_4$ und $R_5$ je Wasserstoff, Halogen oder $C_1$-$C_4$-Alkyl,
$R_6$ Wasserstoff oder Halogen,
$R_7$ ein- oder mehrfach halogeniertes $C_1$-$C_4$-Alkyl und
m die Zahlen 1 oder 2
bedeuten.

19. Schädlingsbekämpfungsmittel gemäss Anspruch 18, welches als aktive Komponente eine Verbindung gemäss einem der Ansprüche 5 bis 12 enthält.

20. Verhindung der Formel I

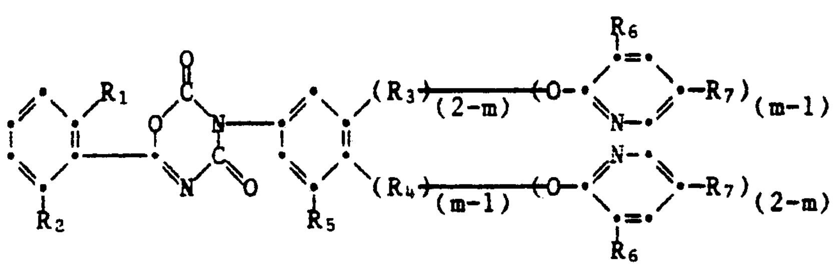

(I),

worin
$R_1$ Halogen, $C_1$-$C_4$-Alkyl, $C_1$-$C_4$-Alkoxy oder $C_1$-$C_4$-Alkylthio,
$R_2$ Wasserstoff, Halogen, $C_1$-$C_4$-Alkyl, $C_1$-$C_4$-Alkoxy oder $C_1$-$C_4$-Alkylthio,
$R_3$ und $R_4$ je Wasserstoff, Halogen oder $C_1$-$C_4$-Alkyl,
$R_5$ Wasserstoff, Halogen, $C_1$-$C_4$-Alkyl oder -$COOR_8$,
$R_6$ Wasserstoff oder Halogen,
$R_7$ ein- oder mehrfach halogeniertes $C_1$-$C_4$-Alkyl,

$R_8$ Wasserstoff oder $C_1$-$C_4$-Alkyl und
m die Zahlen 1 oder 2
bedeuten, zur Verwendung als Schädlingsbekämpfungsmittel an Tieren und Pflanzen.

21. Verbindung der Formel I, worin
$R_1$ Halogen oder $C_1$-$C_4$-Alkyl,
$R_2$ Wasserstoff oder Halogen,
$R_3$ und $R_4$ je Wasserstoff, Halogen oder $C_1$-$C_4$-Alkyl,
$R_5$ Wasserstoff, Halogen, $C_1$-$C_4$-Alkyl oder -$COOR_8$,
$R_6$ Wasserstoff oder Halogen,
$R_7$ ein- oder mehrfach halogeniertes $C_1$-$C_4$-Alkyl,
$R_8$ Wasserstoff oder $C_1$-$C_4$-Alkyl und
m die Zahlen 1 oder 2
bedeuten, zur Verwendung als Schädlingsbekämpfungsmittel an Tieren und Pflanzen.

22. Verbindung der Formel I, worin
$R_1$ Halogen oder $C_1$-$C_4$-Alkyl,
$R_2$ Wasserstoff oder Halogen,
$R_3$, $R_4$ und $R_5$ je Wasserstoff, Halogen oder $C_1$-$C_4$-Alkyl,
$R_6$ Wasserstoff oder Halogen,
$R_7$ ein- oder mehrfach halogeniertes $C_1$-$C_4$-Alkyl und
m die Zahlen 1 oder 2
bedeuten, zur Verwendung als Schädlingsbekämpfungsmittel an Tieren und Pflanzen.

23. Verbindung gemäss einem der Ansprüche 5 bis 12, zur Verwendung als Schädlingsbekämpfungsmittel an Tieren und Pflanzen.

24. Verwendung einer Verbindung der Formel I gemäß Anspruch 1 zur Bekämpfung von pflanzenschädigenden Insekten und Arachniden.

**Patentansprüche** für den Vertragsstaat: AT

1. Schädlingsbekämpfungsmittel, welches als aktive Komponente eine Verbindung der Formel I

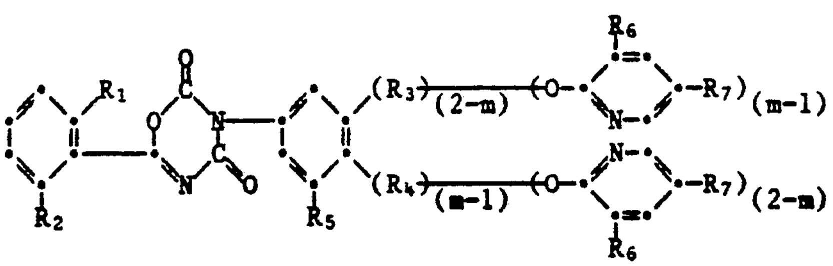

(I),

enthält, worin

$R_1$ Halogen, $C_1$-$C_4$-Alkyl, $C_1$-$C_4$-Alkoxy oder $C_1$-$C_4$-Alkylthio,
$R_2$ Wasserstoff, Halogen, $C_1$-$C_4$-Alkyl, $C_1$-$C_4$-Alkoxy oder $C_1$-$C_4$-Alkylthio,
$R_3$ und $R_4$ je Wasserstoff, Halogen oder $C_1$-$C_4$-Alkyl,
$R_5$ Wasserstoff, Halogen, $C_1$-$C_4$-Alkyl oder -$COOR_8$,
$R_6$ Wasserstoff oder Halogen,
$R_7$ ein- oder mehrfach halogeniertes $C_1$-$C_4$-Alkyl,
$R_8$ Wasserstoff oder $C_1$-$C_4$-Alkyl und
m die Zahlen 1 oder 2
bedeuten, zusammen mit geeigneten Trägern und/oder Zuschlagstoffen.

2. Schädlingsbekämpfungsmittel gemäss Anspruch 1 welches als aktive Komponente eine Verbindung der Formel I enthält, worin

$R_1$ Halogen oder $C_1$-$C_4$-Alkyl,
$R_2$ Wasserstoff oder Halogen,
$R_3$ und $R_4$ je Wasserstoff, Halogen oder $C_1$-$C_4$-Alkyl,
$R_5$ Wasserstoff, Halogen, $C_1$-$C_4$-Alkyl oder -$COOR_8$,
$R_6$ Wasserstoff oder Halogen,
$R_7$ ein- oder mehrfach halogeniertes $C_1$-$C_4$-Alkyl,
$R_8$ Wasserstoff oder $C_1$-$C_4$-Alkyl und

m die Zahlen 1 oder 2
bedeuten.

3. Schädlingsbekämpfungsmittel gemäss Anspruch 2, welches als aktive Komponente eine Verbindung der Formel I enthält, worin

$R_1$ Halogen oder $C_1$-$C_4$-Alkyl,
$R_2$ Wasserstoff oder Halogen,
$R_3$ $R_4$ und $R_5$ je Wasserstoff, Halogen oder $C_1$-$C_4$-Alkyl,
$R_6$ Wasserstoff oder Halogen,
$R_7$ ein- oder mehrfach halogeniertes $C_1$-$C_4$-Alkyl,
m die Zahlen 1 oder 2
bedeuten.

4. Schädlingsbekämpfungsmittel gemäss Anspruch 3, welches als aktive Komponente eine Verbindung der Formel I enthält, worin $R_1$ Halogen, $R_2$, $R_3$, $R_4$ und $R_5$ Wasserstoff oder Halogen, $R_6$ Wasserstoff oder Chlor, $R_7$ ein- oder mehrfach durch Fluor und/oder Chlor substituiertes $C_1$-$C_2$-Alkyl und m die Zahlen 1 oder 2 bedeuten.

5. Schädlingsbekämpfungsmittel gemäss Anspruch 4, welches als aktive Komponente eine Verbindung der Formel I enthält, worin $R_1$ Chlor oder Fluor, $R_2$ Wasserstoff oder Fluor, $R_3$, $R_5$ und $R_6$ Wasserstoff oder Chlor, $R_7$-$CF_3$ oder -$CF_2CFCl_2$ und m die Zahl 1 bedeuten.

6. Schädlingsbekämpfungsmittel gemäss Anspruch 5, welches als aktive Komponente die Verbindung der Formel

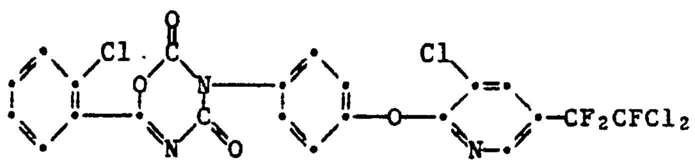

enthält.

7. Schädlingsbekämpfungsmittel gemäss Anspruch 5, welches als aktive Komponente die Verbindung der Formel

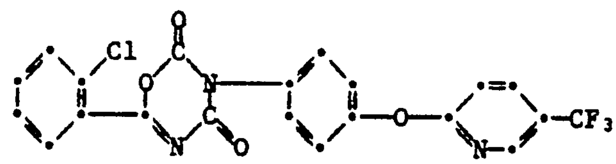

enthält.

8. Schädlingsbekämpfungsmittel gemäss Anspruch 5, welches als aktive Komponente die Verbindung der Formel

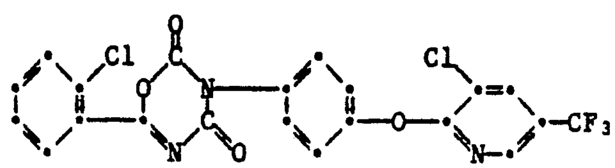

enthält.

9. Schädlingsbekämpfungsmittel gemäss Anspruch 5, welches als aktive Komponente die Verbindung der Formel

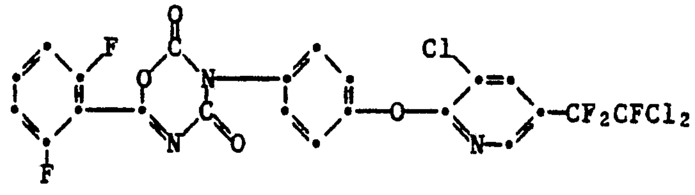

enthält.

10. Schädlingsbekämpfungsmittel gemäss Anspruch 5, welches als aktive Komponente die Verbindung der Formel

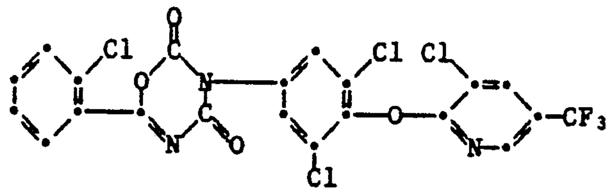

enthält.

11. Schädlingsbekämpfungsmittel gemäss Anspruch 5, welches als aktive Komponente die Verbindung der Formel

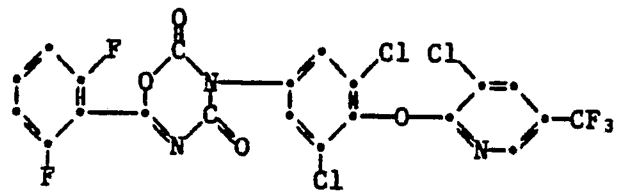

enthält.

12. Schädlingsbekämpfungsmittel gemäss Anspruch 5, welches als aktive Komponente die Verbindung der Formel

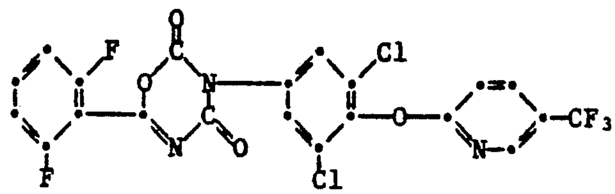

enthalt.

13. Verfahren zur Herstellung einer Verbindung der Formel I

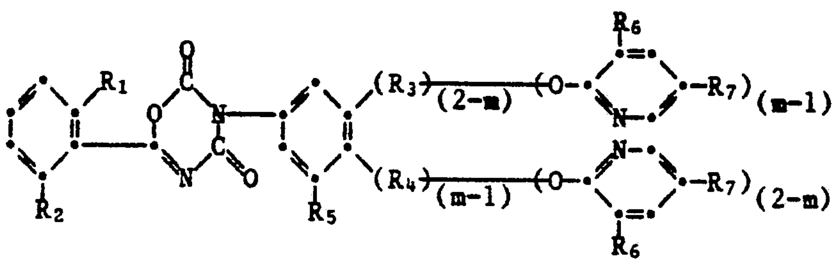

(I),

worin

$R_1$ Halogen, $C_1$-$C_4$-Alkyl, $C_1$-$C_4$-Alkoxy oder $C_1$-$C_4$-Alkylthio,
$R_2$ Wasserstoff, Halogen, $C_1$-$C_4$-Alkyl, $C_1$-$C_4$-Alkoxy oder $C_1$-$C_4$-Alkylthio,
$R_3$ und $R_4$ je Wasserstoff, Halogen oder $C_1$-$C_4$-Alkyl,
$R_5$ Wasserstoff, Halogen, $C_1$-$C_4$-Alkyl oder -$COOR_8$,
$R_6$ Wasserstoff oder Halogen,
$R_7$ ein- oder mehrfach halogeniertes $C_1$-$C_4$-Alkyl,
$R_8$ Wasserstoff oder $C_1$-$C_4$-Alkyl und
m die Zahlen 1 oder 2

bedeuten, dadurch gekennzeichnet, dass man ein Benzoylisocyanat der Formel II

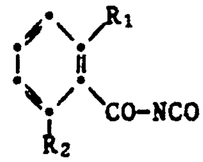

(II)

mit einem Isocyanat der Formel III

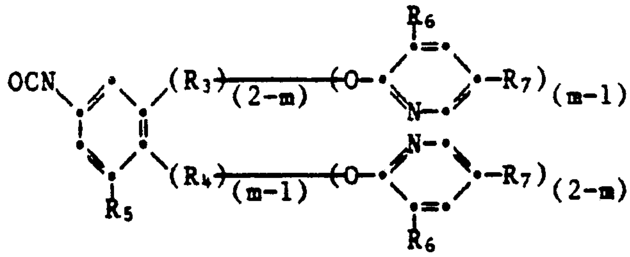

(III)

in bekannter Weise umsetzt, wobei in den Formeln II und III die Symbole $R_1$, $R_2$, $R_3$, $R_4$, $R_5$, $R_6$, $R_7$ und m die für Formel I angegebene Bedeutung haben.

14. Verfahren gemäss Anspruch 13 zur Herstellung einer Verbindung der Formel I, dadurch gekennzeichnet, dass in der Formel I

$R_1$ Halogen oder $C_1$-$C_4$-Alkyl,
$R_2$ Wasserstoff oder Halogen,
$R_3$ und $R_4$ je Wasserstoff, Halogen oder $C_1$-$C_4$-Alkyl,
$R_5$ Wasserstoff, Halogen, $C_1$-$C_4$-Alkyl oder -$COOR_8$,
$R_6$ Wasserstoff oder Halogen,
$R_7$ ein- oder mehrfach halogeniertes $C_1$-$C_4$-Alkyl,
$R_8$ Wasserstoff oder $C_1$-$C_4$-Alkyl und
m die Zahlen 1 oder 2

bedeuten.

15. Verfahren gemäss Anspruch 14 zur Herstellung einer Verbindung der Formel I, dadurch gekennzeichnet, dass in der Formel I

$R_1$ Halogen oder $C_1$-$C_4$-Alkyl,
$R_2$ Wasserstoff oder Halogen,
$R_3$ $R_4$ und $R_5$ je Wasserstoff, Halogen oder $C_1$-$C_4$-Alkyl,
$R_6$ Wasserstoff oder Halogen,
$R_7$ ein- oder mehrfach halogeniertes $C_1$-$C_4$-Alkyl,
m die Zahlen 1 oder 2

bedeuten.

16. Verbindung der Formel I

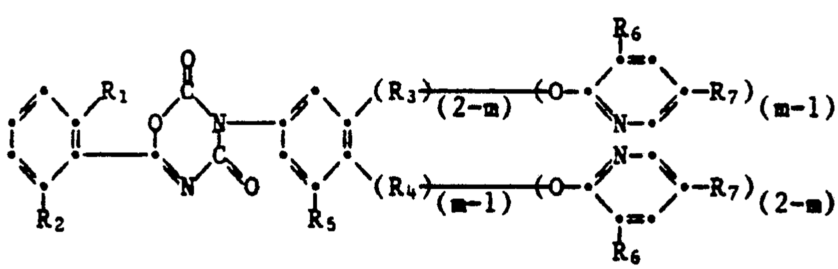

(I),

worin

$R_1$ Halogen, $C_1$-$C_4$-Alkyl, $C_1$-$C_4$-Alkoxy oder $C_1$-$C_4$-Alkylthio,
$R_2$ Wasserstoff, Halogen, $C_1$-$C_4$-Alkyl, $C_1$-$C_4$-Alkoxy oder $C_1$-$C_4$-Alkylthio,
$R_3$ und $R_4$ je Wasserstoff, Halogen oder $C_1$-$C_4$-Alkyl,
$R_5$ Wasserstoff, Halogen, $C_1$-$C_4$-Alkyl oder -$COOR_8$,
$R_6$ Wasserstoff oder Halogen,

$R_7$ ein- oder mehrfach halogeniertes $C_1$-$C_4$-Alkyl,
$R_8$ Wasserstoff oder $C_1$-$C_4$-Alkyl und
m die Zahlen 1 oder 2
bedeuten, zur Verwendung als Schädlinsbekämpfungsmittel an Tieren und Pflanzen.

17. Verwendung einer Verbindung der Formel I, gemäß Anspruch 1 zur Bekämpfung von pflanzenschädigenden Insekten und Arachniden.

**Claims** for the Contracting States: BE, CH, LI, DE, FR, IT, NL

1. Compounds of the formula I

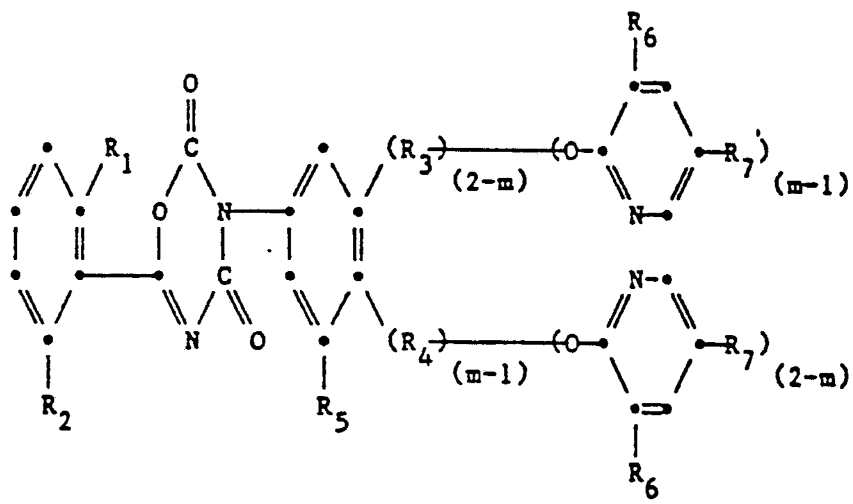

(I)

wherein

$R_1$ is halogen, $C_1$-$C_4$alkyl, $C_1$-$C_4$alkoxy or $C_1$-$C_4$alkylthio,
$R_2$ is hydrogen, halogen, $C_1$-$C_4$alkyl, $C_1$-$C_4$alkoxy or $C_1$-$C_4$-alkylthio,
$R_3$ and $R_4$ are each hydrogen, halogen or $C_1$-$C_4$alkyl,
$R_5$ is hydrogen, halogen, $C_1$-$C_4$alkyl or -$COOR_8$,
$R_6$ is hydrogen or halogen,
$R_7$ is mono- or poly-halogenated $C_1$-$C_4$alkyl,
$R_8$ is hydrogen or $C_1$-$C_4$alkyl, and
m is 1 or 2.

2. Compounds of the formula I according to claim 1, wherein

$R_1$ is halogen or $C_1$-$C_4$alkyl,
$R_2$ is hydrogen, halogen,
$R_3$ and $R_4$ are each hydrogen, halogen or $C_1$-$C_4$alkyl,
$R_5$ is hydrogen, halogen, $C_1$-$C_4$alkyl or -$COOR_8$,
$R_6$ is hydrogen or halogen,
$R_7$ is mono- or poly-halogenated $C_1$-$C_4$alkyl,
$R_8$ is hydrogen or $C_1$-$C_4$alkyl, and
m is 1 or 2.

3. Compounds of the formula I according to claim 2, wherein

$R_1$ is halogen or $C_1$-$C_4$alkyl,
$R_2$ is hydrogen, halogen,
$R_3$ $R_4$ and $R_5$ are each hydrogen, halogen or $C_1$-$C_4$alkyl,
$R_6$ is hydrogen or halogen,
$R_7$ is mono- or poly-halogenated $C_1$-$C_4$alkyl, and
m is 1 or 2.

4. Compounds of the formula I according to claim 3, wherein $R_1$ is halogen, $R_2$, $R_3$, $R_4$ and $R_5$ are hydrogen or halogen, $R_6$ is hydrogen or chlorine, $R_7$ is $C_1$-$C_2$alkyl which is mono- or poly-substituted by fluorine and/or chlorine, and m is 1 or 2.

5. Compounds of the formula I according to claim 4, wherein $R_1$ is chlorine or fluorine, $R_2$ is hydrogen or fluorine, $R_3$, $R_5$ and $R_6$ are hydrogen or chlorine, $R_7$ is $-CF_3$ or $-CF_2CFCl_2$, and m is 1.

6. A compound according to claim 5 of the formula

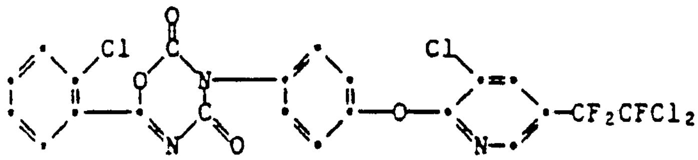

7. A compound according to claim 5 of the formula

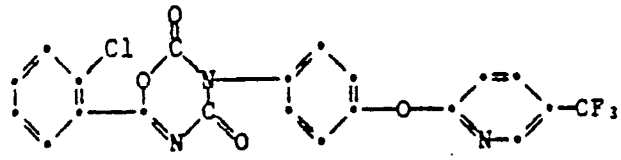

8. A compound according to claim 5 of the formula

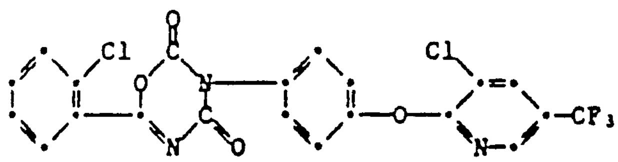

9. A compound according to claim 5 of the formula

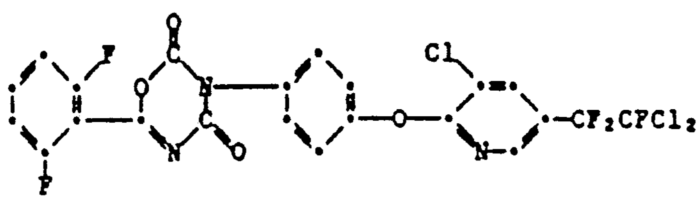

10. A compound according to claim 5 of the formula

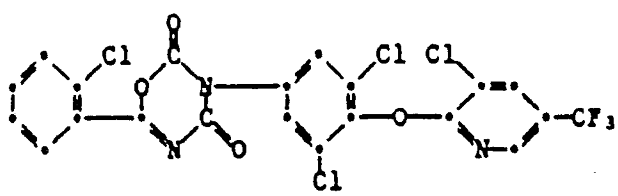

11. A compound according to claim 5 of the formula

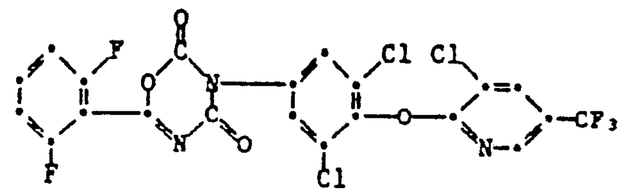

12. A compound according to claim 5 of the formula

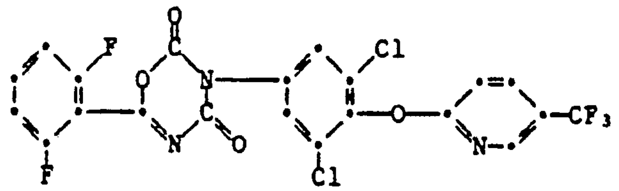

13. A process for producing a compound of the formula I

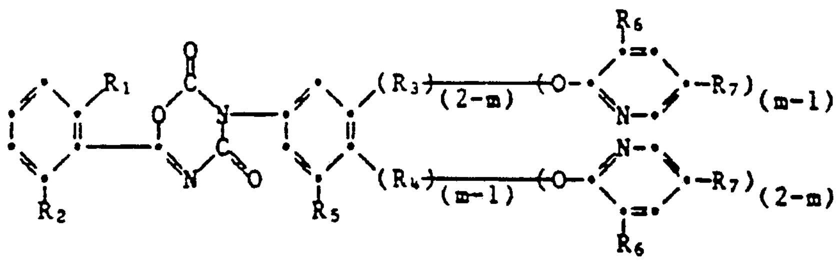

(I)

wherein

$R_1$ is halogen, $C_1$-$C_4$alkyl, $C_1$-$C_4$alkoxy or $C_1$-$C_4$alkylthio,
$R_2$ is hydrogen, halogen, $C_1$-$C_4$alkyl, $C_1$-$C_4$alkoxy or $C_1$-$C_4$-alkylthio,
$R_3$ and $R_4$ are each hydrogen, halogen or $C_1$-$C_4$alkyl,
$R_5$ is hydrogen, halogen, $C_1$-$C_4$alkyl or $-COOR_8$,
$R_6$ is hydrogen or halogen,
$R_7$ is mono- or poly-halogenated $C_1$-$C_4$alkyl,
$R_8$ is hydrogen or $C_1$-$C_4$alkyl, and
m is 1 or 2,

which process comprises reacting, in a known manner, a benzoylisocyanate of the formula II

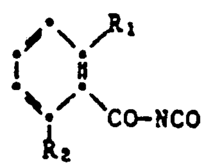

(II)

with an isocyanate of the formula III

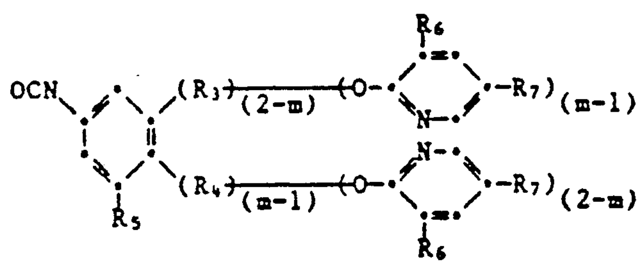

(III),

the symbols $R_1$, $R_2$, $R_3$, $R_4$, $R_5$, $R_6$, $R_7$ and m in the formula II and III having the meanings defined for the formula I.

14. A process according to claim 13 for producing a compound of the formula I, wherein, in the formula I

$R_1$ is halogen or $C_1$-$C_4$alkyl,
$R_2$ is hydrogen, halogen,
$R_3$ and $R_4$ are each hydrogen, halogen or $C_1$-$C_4$alkyl,
$R_5$ is hydrogen, halogen, $C_1$-$C_4$alkyl or -$COOR_8$,
$R_6$ is hydrogen or halogen,
$R_7$ is mono- or poly-halogenated $C_1$-$C_4$alkyl,
$R_8$ is hydrogen or $C_1$-$C_4$alkyl, and
m is 1 or 2.

15. A process according to claim 14 for producing a compound of the formula I, wherein, in the formula I

$R_1$ is halogen or $C_1$-$C_4$alkyl,
$R_2$ is hydrogen, halogen,
$R_3$ $R_4$ and $R_5$ are each hydrogen, halogen or $C_1$-$C_4$alkyl,
$R_6$ is hydrogen or halogen,
$R_7$ is mono- or poly-halogenated $C_1$-$C_4$alkyl, and
m is 1 or 2.

16. A pesticidal composition which contains, as active ingredient, a compound of the formula I

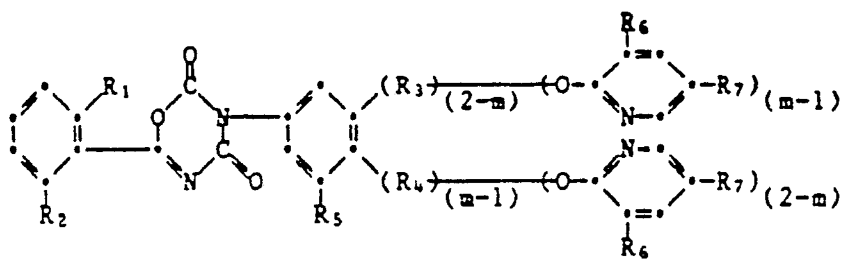

(I)

wherein

$R_1$ is halogen, $C_1$-$C_4$alkyl, $C_1$-$C_4$alkoxy or $C_1$-$C_4$alkylthio,
$R_2$ is hydrogen, halogen, $C_1$-$C_4$alkyl, $C_1$-$C_4$alkoxy or $C_1$-$C_4$-alkylthio,
$R_3$ and $R_4$ are each hydrogen, halogen or $C_1$-$C_4$alkyl,
$R_5$ is hydrogen, halogen, $C_1$-$C_4$alkyl or -$COOR_8$,
$R_6$ is hydrogen or halogen,
$R_7$ is mono- or poly-halogenated $C_1$-$C_4$alkyl,
$R_8$ is hydrogen or $C_1$-$C_4$alkyl, and
m is 1 or 2,
together with suitable carriers and/or additives.

17. A pesticidal composition according to claim 16, which contains, as active ingredient, a compound of the formula I wherein

$R_1$ is halogen or $C_1$-$C_4$alkyl,
$R_2$ is hydrogen, halogen,
$R_3$ and $R_4$ are each hydrogen, halogen or $C_1$-$C_4$alkyl,
$R_5$ is hydrogen, halogen, $C_1$-$C_4$alkyl or -$COOR_8$,
$R_6$ is hydrogen or halogen,
$R_7$ is mono- or poly-halogenated $C_1$-$C_4$alkyl,
$R_8$ is hydrogen or $C_1$-$C_4$alkyl, and
m is 1 or 2.

18. A pesticidal composition according to claim 17, which contains, as active ingredient, a compound of the formula I wherein

$R_1$ is halogen or $C_1$-$C_4$alkyl,
$R_2$ is hydrogen, halogen,
$R_3$
$R_4$ and $R_5$ are each hydrogen, halogen or $C_1$-$C_4$alkyl,

$R_6$ is hydrogen or halogen,
$R_7$ is mono- or poly-halogenated $C_1$-$C_4$alkyl, and
m is 1 or 2.

19. A pesticidal composition according to claim 18, which contains, as active ingredient, a compound according to any one of claims 5 to 12.

20. A compound of the formula I

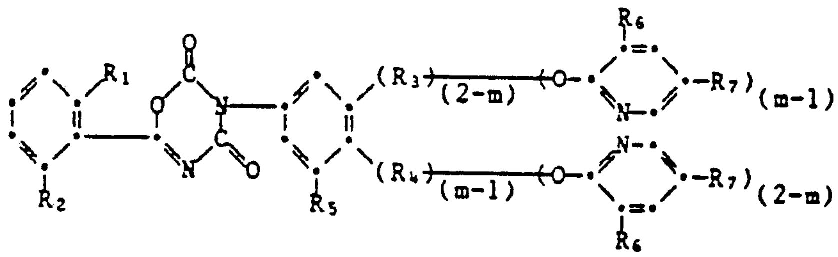

(I)

wherein

$R_1$ is halogen, $C_1$-$C_4$alkyl, $C_1$-$C_4$alkoxy or $C_1$-$C_4$alkylthio,
$R_2$ is hydrogen, halogen, $C_1$-$C_4$alkyl, $C_1$-$C_4$alkoxy or $C_1$-$C_4$-alkylthio,
$R_3$ and $R_4$ are each hydrogen, halogen or $C_1$-$C_4$alkyl,
$R_5$ is hydrogen, halogen, $C_1$-$C_4$alkyl or $-COOR_8$,
$R_6$ is hydrogen or halogen,
$R_7$ is mono- or poly-halogenated $C_1$-$C_4$alkyl,
$R_8$ is hydrogen or $C_1$-$C_4$alkyl, and
m is 1 or 2,
for use as a pesticidal composition on animals and plants.

21. A compound of the formula I wherein

$R_1$ is halogen or $C_1$-$C_4$alkyl,
$R_2$ is hydrogen, halogen,
$R_3$ and $R_4$ are each hydrogen, halogen or $C_1$-$C_4$alkyl,
$R_5$ is hydrogen, halogen, $C_1$-$C_4$alkyl or $-COOR_8$,
$R_6$ is hydrogen or halogen,
$R_7$ is mono- or poly-halogenated $C_1$-$C_4$alkyl,
$R_8$ is hydrogen or $C_1$-$C_4$alkyl, and
m is 1 or 2,
for use as a pesticidal composition on animals and plants.

22. A compound of the formula I wherein

$R_1$ is halogen or $C_1$-$C_4$alkyl,
$R_2$ is hydrogen, halogen,
$R_3$ $R_4$ and $R_5$ are each hydrogen, halogen or $C_1$-$C_4$alkyl,
$R_6$ is hydrogen or halogen,
$R_7$ is mono- or poly-halogenated $C_1$-$C_4$alkyl, and
m is 1 or 2,
for use as a pesticidal composition on animals and plants.

23. A compound according to any one of claims 5 to 12 for use as a pesticidal composition on animals and plants.

24. The use of a compound of the formula I according to claim 1 for controlling plant-damaging insects and arachnids.

**Claims** for the Contracting State: AT

1. A pesticidal composition which contains, as active ingredient, a compound of the formula I

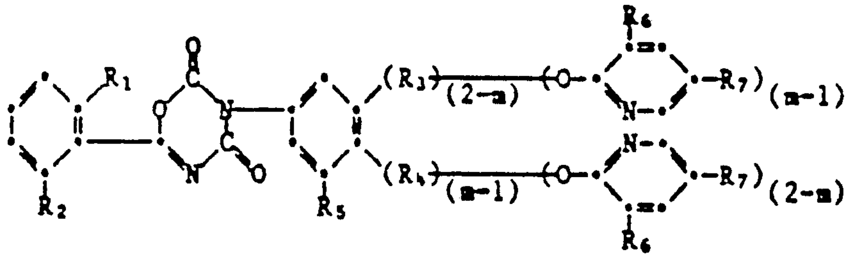

(I)

wherein

$R_1$ is halogen, $C_1$-$C_4$alkyl, $C_1$-$C_4$alkoxy or $C_1$-$C_4$alkylthio,
$R_2$ is hydrogen, halogen, $C_1$-$C_4$alkyl, $C_1$-$C_4$alkoxy or $C_1$-$C_4$-alkylthio,
$R_3$ and $R_4$ are each hydrogen, halogen or $C_1$-$C_4$alkyl,
$R_5$ is hydrogen, halogen, $C_1$-$C_4$alkyl or -$COOR_8$,
$R_6$ is hydrogen or halogen,
$R_7$ is mono- or poly-halogenated $C_1$-$C_4$alkyl,
$R_8$ is hydrogen or $C_1$-$C_4$alkyl, and
m is 1 or 2,
together with suitable carriers and/or additives.

2. A pesticidal composition according to claim 1, which contains, as active ingredient, a compound of the formula I wherein

$R_1$ is halogen or $C_1$-$C_4$alkyl,
$R_2$ is hydrogen, halogen,
$R_3$ and $R_4$ are each hydrogen, halogen or $C_1$-$C_4$alkyl,
$R_5$ is hydrogen, halogen, $C_1$-$C_4$alkyl or -$COOR_8$,
$R_6$ is hydrogen or halogen,
$R_7$ is mono- or poly-halogenated $C_1$-$C_4$alkyl,
$R_8$ is hydrogen or $C_1$-$C_4$alkyl, and
m is 1 or 2.

3. A pesticidal composition according to claim 2, which contains, as active ingredient, a compound of the formula I wherein

$R_1$ is halogen or $C_1$-$C_4$alkyl,
$R_2$ is hydrogen, halogen,
$R_3$ $R_4$ and $R_5$ are each hydrogen, halogen or $C_1$-$C_4$alkyl,
$R_6$ is hydrogen or halogen,
$R_7$ is mono- or poly-halogenated $C_1$-$C_4$alkyl, and
m is 1 or 2.

4. A pesticidal composition according to claim 3, which contains, as active ingredient, a compound of the formula I wherein $R_1$ is halogen, $R_2$, $R_3$, $R_4$ and $R_5$ are hydrogen or halogen, $R_6$ is hydrogen or chlorine, $R_7$ is $C_1$-$C_2$alkyl which is mono- or poly-substituted by fluorine and/or chlorine, and m is 1 or 2.

5. A pesticidal composition according to claim 4, which contains, as active ingredient, a compound of the formula I wherein $R_1$ is chlorine or fluorine, $R_2$ is hydrogen or fluorine, $R_3$, $R_5$ and $R_6$ are hydrogen or chlorine, $R_7$ is -$CF_3$ or -$CF_2CFCl_2$, and m is 1.

6. A pesticidal composition according to claim 5, which contains, as active ingredient, a compound of the formula

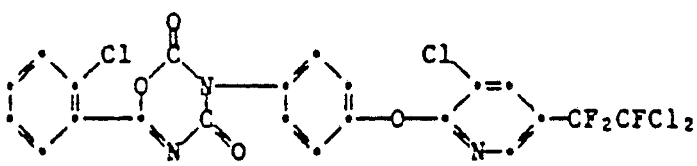

7. A pesticidal composition according to claim 5, which contains, as active ingredient, a compound of the formula

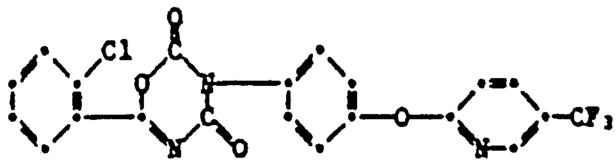

8. A pesticidal composition according to claim 5, which contains, as active ingredient, a compound of the formula

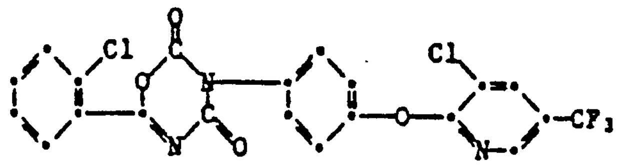

9. A pesticidal composition according to claim 5, which contains, as active ingredient, a compound of the formula

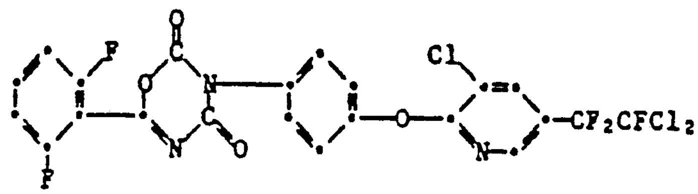

10. A pesticidal composition according to claim 5, which contains, as active ingredient, a compound of the formula

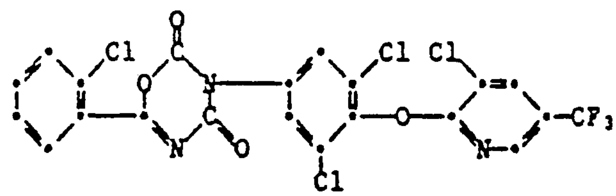

11. A pesticidal composition according to claim 5, which contains, as active ingredient, a compound of the formula

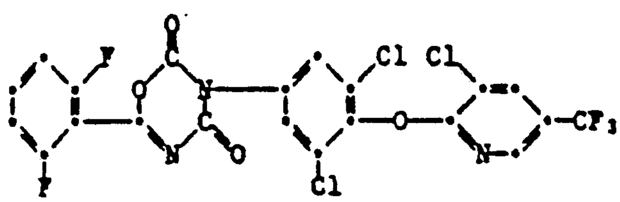

12. A pesticidal composition according to claim 5, which contains, as active ingredient, a compound of the formula

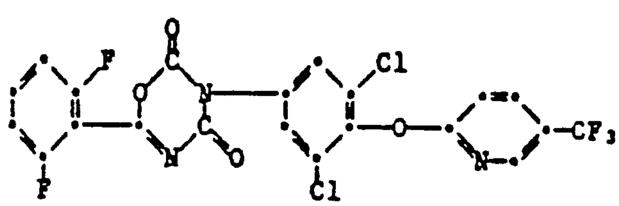

13. A process for producing a compound of the formula I

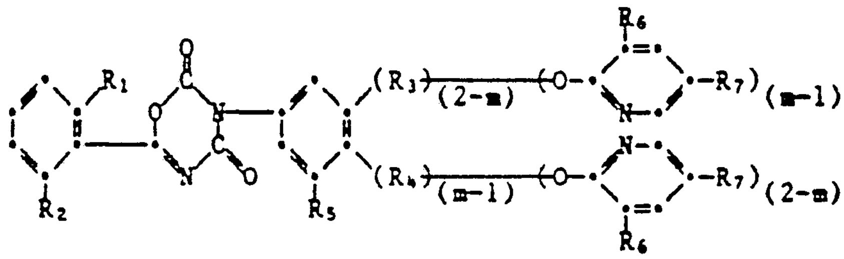

(I)

wherein

$R_1$ is halogen, $C_1$-$C_4$alkyl, $C_1$-$C_4$alkoxy or $C_1$-$C_4$alkylthio,
$R_2$ is hydrogen, halogen, $C_1$-$C_4$alkyl, $C_1$-$C_4$alkoxy or $C_1$-$C_4$-alkylthio,
$R_3$ and $R_4$ are each hydrogen, halogen or $C_1$-$C_4$alkyl,
$R_5$ is hydrogen, halogen, $C_1$-$C_4$alkyl or $-COOR_8$,
$R_6$ is hydrogen or halogen,
$R_7$ is mono- or poly-halogenated $C_1$-$C_4$alkyl,
$R_8$ is hydrogen or $C_1$-$C_4$alkyl, and
m is 1 or 2,
which process comprises reacting, in a known manner, a benzoylisocyanate of the formula II

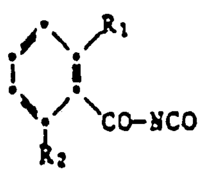

(II)

with an isocyanate of the formula III

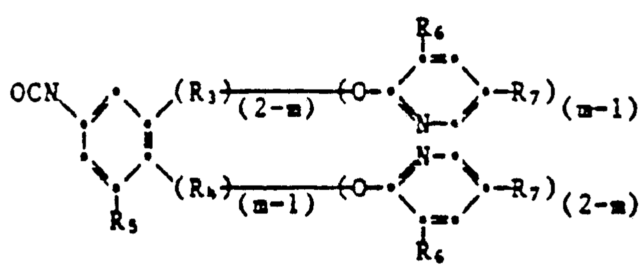

(III),

the symbols $R_1$, $R_2$, $R_3$, $R_4$, $R_5$, $R_6$, $R_7$ and m in the formulae II and III having the meanings defined for the formula I.

14. A process according to claim 13 for producing a compound of the formula I, wherein, in the formula I

$R_1$ is halogen or $C_1$-$C_4$alkyl,
$R_2$ is hydrogen, halogen,
$R_3$ and $R_4$ are each hydrogen, halogen or $C_1$-$C_4$alkyl,
$R_5$ is hydrogen, halogen, $C_1$-$C_4$alkyl or $-COOR_8$,
$R_6$ is hydrogen or halogen,
$R_7$ is mono- or poly-halogenated $C_1$-$C_4$alkyl,
$R_8$ is hydrogen or $C_1$-$C_4$alkyl, and
m is 1 or 2.

15. A process according to claim 14 for producing a compound of the formula I, wherein, in the formula I

$R_1$ is halogen or $C_1$-$C_4$alkyl,
$R_2$ is hydrogen, halogen,
$R_3$ $R_4$ and $R_5$ are each hydrogen, halogen or $C_1$-$C_4$alkyl,
$R_6$ is hydrogen or halogen,

$R_7$ is mono- or poly-halogenated $C_1$-$C_4$alkyl, and
m is 1 or 2.

16. A compound of the formula I

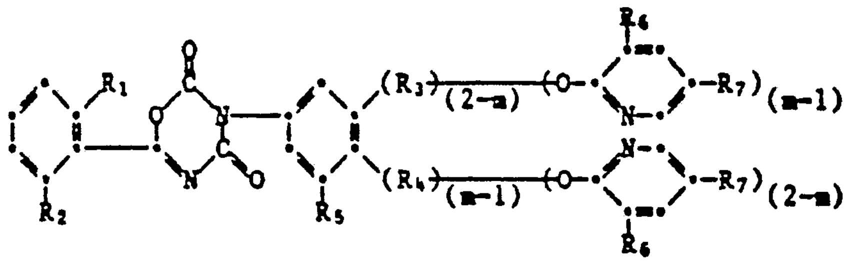

(I)

wherein

$R_1$ is halogen, $C_1$-$C_4$alkyl, $C_1$-$C_4$alkoxy or $C_1$-$C_4$alkylthio,
$R_2$ is hydrogen, halogen, $C_1$-$C_4$alkyl, $C_1$-$C_4$alkoxy or $C_1$-$C_4$-alkylthio,
$R_3$ and $R_4$ are each hydrogen, halogen or $C_1$-$C_4$alkyl,
$R_5$ is hydrogen, halogen, $C_1$-$C_4$alkyl or $-COOR_8$,
$R_6$ is hydrogen or halogen,
$R_7$ is mono- or poly-halogenated $C_1$-$C_4$alkyl,
$R_8$ is hydrogen or $C_1$-$C_4$alkyl, and
m is 1 or 2,
for use as a pesticidal composition on animals and plants.

17. The use of a compound of the formula I according to claim 1 for controlling plant-damaging insects and arachnids.

**Revendications** pour les Etats Contractants: BE, CH, LI, DE, IT, NL

1. Composés de formule I

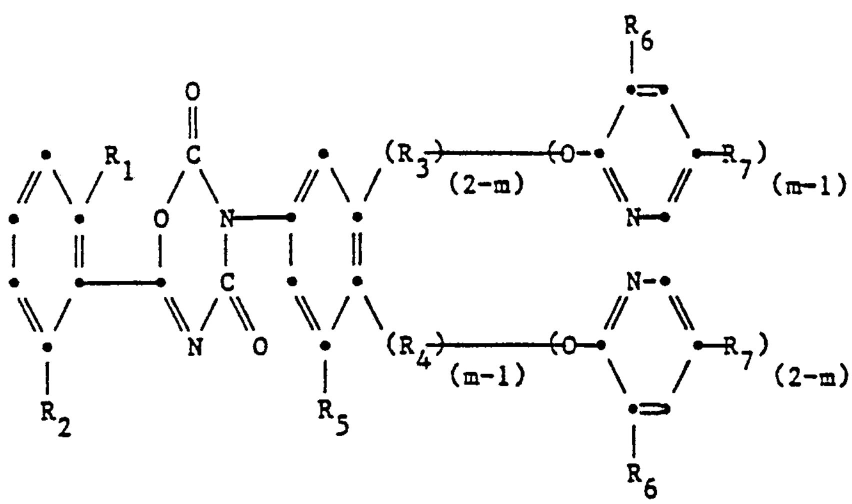

(I),

dans laquelle

$R_1$ représente un halogène, un groupe alkyle en $C_1$-$C_4$, alcoxy en $C_1$-$C_4$ ou alkylthio en $C_1$-$C_4$,
$R_2$ représente l'hydrogène, un halogène, un groupe alkyle en $C_1$-$C_4$, alcoxy en $C_1$-$C_4$ ou alkylthio en $C_1$-$C_4$,
$R_3$ et $R_4$ représentent chacun l'hydrogène, un halogène ou un groupe alkyle en $C_1$-$C_4$,
$R_5$ représente l'hydrogène, un halogène, un groupe alkyle en $C_1$-$C_4$ ou $-COOR_8$,
$R_6$ représente l'hydrogène ou un halogène,
$R_7$ représente un groupe alkyle en $C_1$-$C_4$ mono- ou polyhalogéné,
$R_8$ représente l'hydrogène ou un groupe alkyle en $C_1$-$C_4$ et
m est égal à 1 ou 2.

2. Composés de formule I selon la revendication 1, dans lesquels

$R_1$ représente un halogène, un groupe alkyle en $C_1$-$C_4$,
$R_2$ représente l'hydrogène, un halogène,
$R_3$
et $R_4$ représentent chacun l'hydrogène, un halogène ou un groupe alkyle en $C_1$-$C_4$,
$R_5$ représente l'hydrogène, un halogène, un groupe alkyle en $C_1$-$C_4$ ou $-COOR_8$,
$R_6$ représente l'hydrogène ou un halogène,
$R_7$ représente un groupe alkyle en $C_1$-$C_4$ mono- ou polyhalogéné,
$R_8$ représente l'hydrogène ou un groupe alkyle en $C_1$-$C_4$ et
m est égal à 1 ou 2.

3. Composés de formule I selon la revendication 2, dans lesquels

$R_1$ représente un halogène ou un groupe alkyle en $C_1$-$C_4$,
$R_2$ représente l'hydrogène, un halogène,
$R_3$ $R_4$ et $R_5$ représentent chacun l'hydrogène, un halogène ou un groupe alkyle en $C_1$-$C_4$,
$R_6$ représente l'hydrogène ou un halogène,
$R_7$ représente un groupe alkyle en $C_1$-$C_4$ mono- ou polyhalogéné,
m est égal à 1 ou 2.

4. Composés de formule I selon la revendication 3, dans lesquels $R_1$ représente un halogène, $R_2$, $R_3$, $R_4$, et $R_5$ représentent l'hydrogène ou un halogène, $R_6$ représente l'hydrogène ou le chlore, $R_7$ représente un groupe alkyle en $C_1$-$C_2$ mono- ou poly-substitué par le fluor et/ou le chlore, et m est égal à 1 ou 2.

5. Composés de formule I selon la revendication 4, dans lesquels $R_1$ représente le chlore ou le fluor, $R_2$ représente l'hydrogène ou le fluor, $R_3$, $R_5$, et $R_6$ représentent l'hydrogène ou le chlore, $R_7$ représente $-CF_3$ ou $-CF_2CFCl_2$ et m est égal à 1.

6. Composé selon la revendication 5, de formule

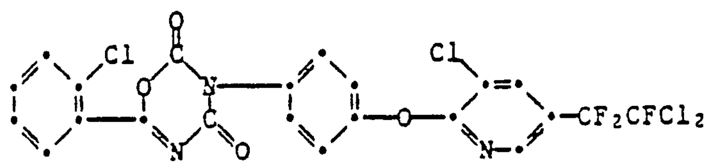

7. Composé selon la revendication 5, de formule

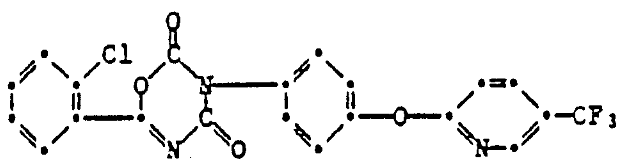

8. Composé selon la revendication 5, de formule

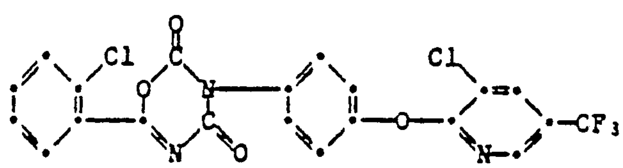

9. Composé selon la revendication 5, de formule

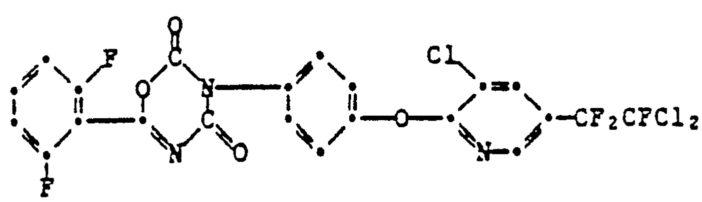

10. Composé selon la revendication 5, de formule

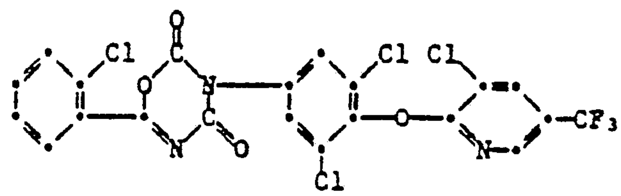

11. Composé selon la revendication 5, de formule

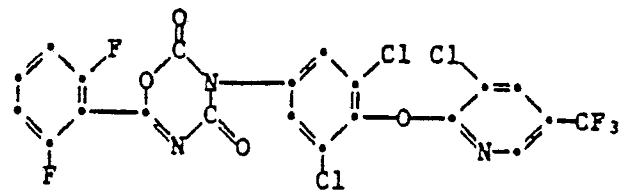

12. Composé selon la revendication 5, de formule

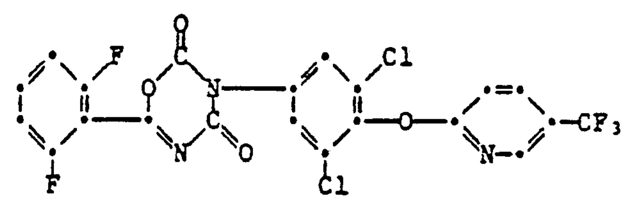

13. Procédé de préparation d'un composé de formule I

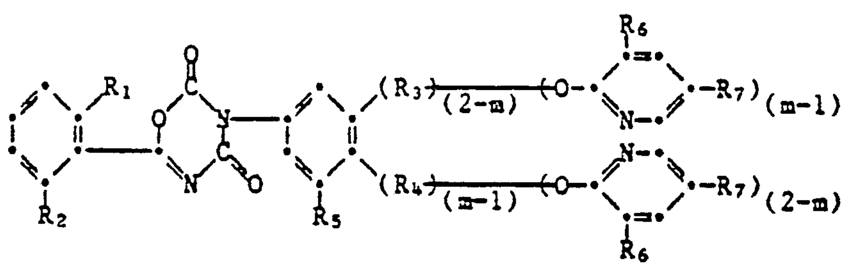 (I),

dans laquelle

$R_1$ représente un halogène, un groupe alkyle en $C_1$-$C_4$, alcoxy en $C_1$-$C_4$ ou alkylthio en $C_1$-$C_4$,
$R_2$ représente l'hydrogène, un halogène, un groupe alkyle en $C_1$-$C_4$, alcoxy en $C_1$-$C_4$ ou alkylthio en $C_1$-$C_4$,
$R_3$ et $R_4$ représentent chacun l'hydrogène, un halogène ou un groupe alkyle en $C_1$-$C_4$,
$R_5$ représente l'hydrogène, un halogène, un groupe alkyle en $C_1$-$C_4$ ou -$COOR_8$,
$R_6$ représente l'hydrogène ou un halogène,
$R_7$ représente un groupe alkyle en $C_1$-$C_4$ mono- ou polyhalogéné,
$R_8$ représente l'hydrogène ou un groupe alkyle en $C_1$-$C_4$ et
m est égal à 1 ou 2,
caractérisé en ce qué l'on fait réagir de manière connue un isocyanate de benzoyle de formule II

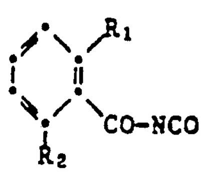 (II)

avec un isocyanate de formule III

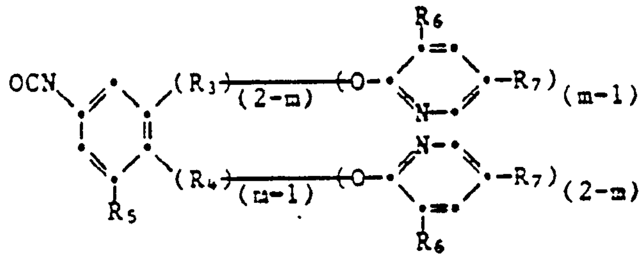

(III)

les symboles $R_1$, $R_2$, $R_3$, $R_4$, $R_5$, $R_6$, $R_7$ et m ayant dans les formules II et III les significations indiquées en référence à la formule I.

14. Procédé selon la revendication 13 pour la préparation d'un composé de formule I, caractérisé en ce que, dans la formule I,

$R_1$ représente un halogène, un groupe alkyle en $C_1$-$C_4$,
$R_2$ représente l'hydrogène, un halogène,
$R_3$ et $R_4$ représentent chacun l'hydrogène, un halogène ou un groupe alkyle en $C_1$-$C_4$,
$R_5$ représente l'hydrogène, un halogène, un groupe alkyle en $C_1$-$C_4$ ou $-COOR_8$,
$R_6$ représente l'hydrogène ou un halogène,
$R_7$ représente un groupe alkyle en $C_1$-$C_4$ mono- ou polyhalogéné,
$R_8$ représente l'hydrogène ou un groupe alkyle en $C_1$-$C_4$ et
m est égal à 1 ou 2.

15. Procédé selon la revendication 14 pour la préparation d'un composé de formule I, caractérisé en ce que, dans la formule I,

$R_1$ représente un halogène ou un groupe alkyle en $C_1$-$C_4$,
$R_2$ représente l'hydrogène, un halogène,
$R_3$ $R_4$ et $R_5$ représentent chacun l'hydrogène, un halogène ou un groupe alkyle en $C_1$-$C_4$,
$R_6$ représente l'hydrogène ou un halogène,
$R_7$ représente un groupe alkyle en $C_1$-$C_4$ mono- ou polyhalogéné,
m est égal à 1 ou 2.

16. Produit pesticide contenant en tant que composant actif un composé de formule I

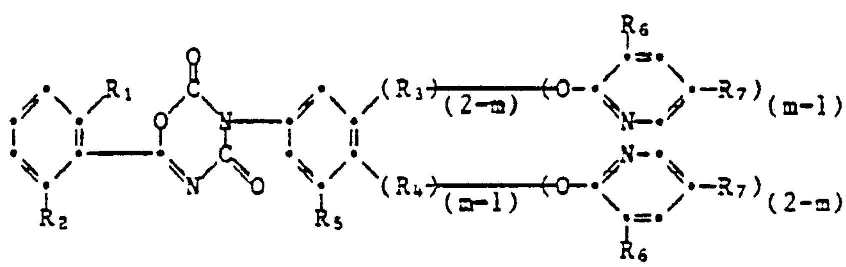

(I),

dans laquelle

$R_1$ représente un halogène, un groupe alkyle en $C_1$-$C_4$, alcoxy en $C_1$-$C_4$ ou alkylthio en $C_1$-$C_4$,
$R_2$ représente l'hydrogène, un halogène, un groupe alkyle en $C_1$-$C_4$, alcoxy en $C_1$-$C_4$ ou alkylthio en $C_1$-$C_4$,
$R_3$ et $R_4$ représentent chacun l'hydrogène, un halogène ou un groupe alkyle en $C_1$-$C_4$,
$R_5$ représente l'hydrogène, un halogène, un groupe alkyle en $C_1$-$C_4$ ou $-COOR_8$,
$R_6$ représente l'hydrogène ou un halogène,
$R_7$ représente un groupe alkyle en $C_1$-$C_4$ mono- ou polyhalogéné,
$R_8$ représente l'hydrogène ou un groupe alkyle en $C_1$-$C_4$ et
m est égal à 1 ou 2,

avec des véhicules et/ou additifs appropriés.

17. Produit pesticide selon la revendication 16, contenant en tant que composant actif un composé de formule I dans laquelle

$R_1$ représente un halogène, un groupe alkyle en $C_1$-$C_4$,
$R_2$ représente l'hydrogène, un halogène,

$R_3$ et $R_4$ représentent chacun l'hydrogène, un halogène ou un groupe alkyle en $C_1$-$C_4$,
$R_5$ représente l'hydrogène, un halogène, un groupe alkyle en $C_1$-$C_4$ ou -$COOR_8$,
$R_6$ représente l'hydrogène ou un halogène,
$R_7$ représente un groupe alkyle en $C_1$-$C_4$ mono- ou polyhalogéné,
$R_8$ représente l'hydrogène ou un groupe alkyle en $C_1$-$C_4$ et
m est égal à 1 ou 2.

18. Produit pesticide selon la revendication 17, contenant en tant que composant actif un composé de formule I dans laquelle

$R_1$ représente un halogène ou un groupe alkyle en $C_1$-$C_4$,
$R_2$ représente l'hydrogène, un halogène,
$R_3$ $R_4$ et $R_5$ représentent chacun l'hydrogène, un halogène ou un groupe alkyle en $C_1$-$C_4$,
$R_6$ représente l'hydrogène ou un halogène,
$R_7$ représente un groupe alkyle en $C_1$-$C_4$ mono- ou polyhalogéné,
m est égal à 1 ou 2.

19. Produit pesticide selon la revendication 18, contenant en tant que composant actif un composé selon l'une des revendications 5 à 12.

20. Composé de formule I

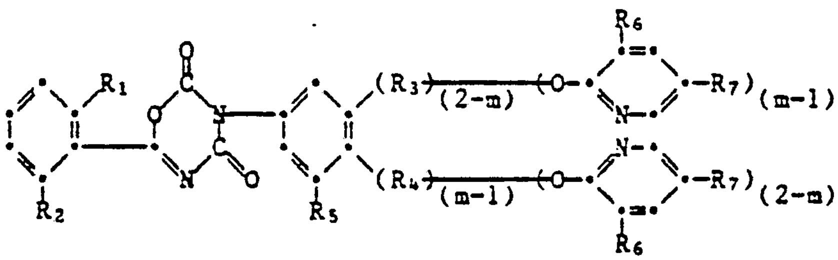

(I),

dans laquelle

$R_1$ représente un halogène, un groupe alkyle en $C_1$-$C_4$, alcoxy en $C_1$-$C_4$ ou alkylthio en $C_1$-$C_4$,
$R_2$ représente l'hydrogène, un halogène, un groupe alkyle en $C_1$-$C_4$, alcoxy en $C_1$-$C_4$ ou alkylthio en $C_1$-$C_4$,
$R_3$ et $R_4$ représentent chacun l'hydrogène, un halogène ou un groupe alkyle en $C_1$-$C_4$,
$R_5$ représente l'hydrogène, un halogène, un groupe alkyle en $C_1$-$C_4$ ou -$COOR_8$,
$R_6$ représente l'hydrogène ou un halogène,
$R_7$ représente un groupe alkyle en $C_1$-$C_4$ mono- ou polyhalogéné,
$R_8$ représente l'hydrogène ou un groupe alkyle en $C_1$-$C_4$ et
m est égal à 1 ou 2,
pour l'utilisation en tant que produit pesticide sur des animaux et des végétaux.

21. Composé de formule I dans laquelle

$R_1$ représente un halogène, un groupe alkyle en $C_1$-$C_4$,
$R_2$
représente l'hydrogène, un halogène,
$R_3$ et $R_4$ représentent chacun l'hydrogène, un halogène ou un groupe alkyle en $C_1$-$C_4$,
$R_5$ représente l'hydrogène, un halogène, un groupe alkyle en $C_1$-$C_4$ ou -$COOR_8$,
$R_6$ représente l'hydrogène ou un halogène,
$R_7$ représente un groupe alkyle en $C_1$-$C_4$ mono- ou polyhalogéné,
$R_8$ représente l'hydrogène ou un groupe alkyle en $C_1$-$C_4$ et
m est égal à 1 ou 2,
pour l'utilisation en tant que produit pesticide sur des animaux et des végétaux.

22. Composé de formule I dans laquelle

$R_1$ représente un halogène ou un groupe alkyle en $C_1$-$C_4$,
$R_2$ représente l'hydrogène, un halogène,
$R_3$ $R_4$ et $R_5$ représentent chacun l'hydrogène, un halogène ou un groupe alkyle en $C_1$-$C_4$,
$R_6$ représente l'hydrogène ou un halogène,
$R_7$ représente un groupe alkyle en $C_1$-$C_4$ mono- ou polyhalogéné,
m est égal à 1 ou 2,
pour l'utilisation en tant que produit pesticide sur des animaux et des végétaux.

23. Composé selon l'une des revendications 5 à 12 pour l'utilisation en tant que produit pesticide sur des

animaux et des végétaux.

24. Utilisation d'un composé de formule I selon la revendication 1 dans la lutte contre les insectes et les arachnides nuisibles pour les végétaux.

**Revendications** pour l'Etat Contractant: AT

1. Produit pesticide contenant en tant que composant actif un composé de formule I

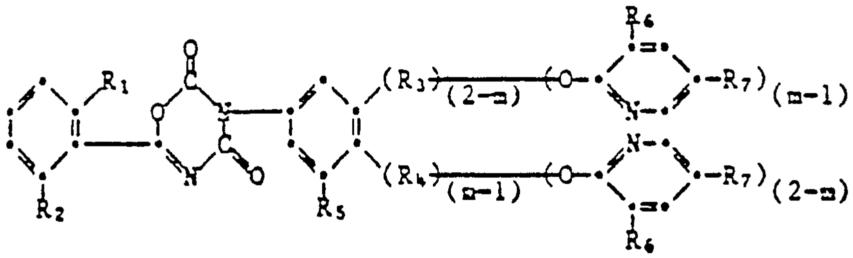

(I),

dans laquelle

$R_1$ représente un halogène, un groupe alkyle en $C_1$-$C_4$, alcoxy en $C_1$-$C_4$ ou alkylthio en $C_1$-$C_4$,
$R_2$ représente l'hydrogène, un halogène, un groupe alkyle en $C_1$-$C_4$, alcoxy en $C_1$-$C_4$ ou alkylthio en $C_1$-$C_4$,
$R_3$ et $R_4$ représentent chacun l'hydrogène, un halogène ou un groupe alkyle en $C_1$-$C_4$,
$R_5$ représente l'hydrogène, un halogène, un groupe alkyle en $C_1$-$C_4$ ou -$COOR_8$,
$R_6$ représente l'hydrogène ou un halogène,
$R_7$ représente un groupe alkyle en $C_1$-$C_4$ mono- ou polyhalogéné,
$R_8$ représente l'hydrogène ou un groupe alkyle en $C_1$-$C_4$ et
m est égal à 1 ou 2,
avec des véhicules et/ou additifs appropriés.

2. Produit pesticide selon la revendication 1, contenant en tant que composant actif un composé de formule I dans laquelle

$R_1$ représente un halogène, un groupe alkyle en $C_1$-$C_4$,
$R_2$ représente l'hydrogène, un halogène,
$R_3$ et $R_4$ représentent chacun l'hydrogène, un halogène ou un groupe alkyle en $C_1$-$C_4$,
$R_5$ représente l'hydrogène, un halogène, un groupe alkyle en $C_1$-$C_4$ ou -$COOR_8$,
$R_6$ représente l'hydrogène ou un halogène,
$R_7$ représente un groupe alkyle en $C_1$-$C_4$ mono- ou polyhalogéné,
$R_8$ représente l'hydrogène ou un groupe alkyle en $C_1$-$C_4$ et
m est égal à 1 ou 2.

3. Produit pesticide selon la revendication 2, contenant en tant que composant actif un composé de formule I dans laquelle

$R_1$ représente un halogène ou un groupe alkyle en $C_1$-$C_4$,
$R_2$ représente l'hydrogène, un halogène,
$R_3$ $R_4$ et $R_5$ représentent chacun l'hydrogène, un halogène ou un groupe alkyle en $C_1$-$C_4$,
$R_6$ représente l'hydrogène ou un halogène,
$R_7$ représente un groupe alkyle en $C_1$-$C_4$ mono- ou polyhalogéné,
m est égal à 1 ou 2.

4. Produit pesticide selon la revendication 3, contenant en tant que composé actif un composé de formule I dans laquelle $R_1$ représente un halogène, $R_2$, $R_3$, $R_4$ et $R_5$ représentent l'hydrogène ou des halogènes, $R_6$ représente l'hydrogène ou le chlore, $R_7$ représente un groupe alkyle en $C_1$-$C_2$ mono- ou poly-substitué par le fluor et/ou le chlore, et m est égal à 1 ou 2.

5. Produit pesticide selon la revendication 4, contenant en tant que composant actif un composé de formule I dans laquelle $R_1$ représente le chlore ou le fluor, $R_2$ l'hydrogène ou le fluor, $R_3$, $R_5$ et $R_6$ représentent l'hydrogène ou le chlore, $R_7$ un groupe -$CF_3$ ou -$CF_2CFCl_2$ et m est égal à 1.

6. Produit pesticide selon la revendication 5, contenant en tant que composant actif le conposé de formule

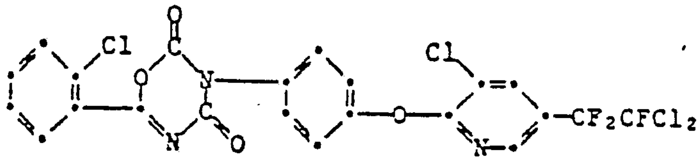

7. Produit pesticide selon la revendication 5, contenant en tant que composant actif le composé de formule

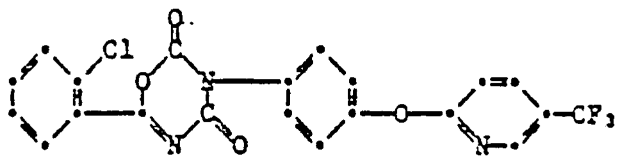

8. Produit pesticide selon la revendication 5, contenant en tant que composant actif le composé de formule

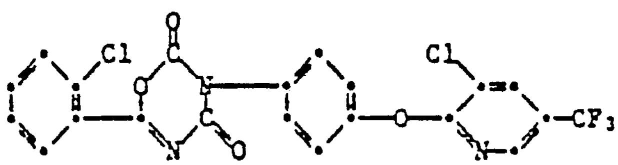

9. Produit pesticide selon la revendication 5, contenant en tant que composant actif le composé de formule

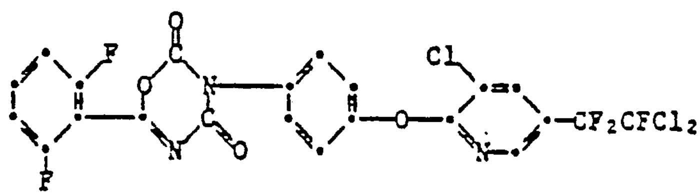

10. Produit pesticide selon la revendication 5, contenant en tant que composant actif le composé dé formule

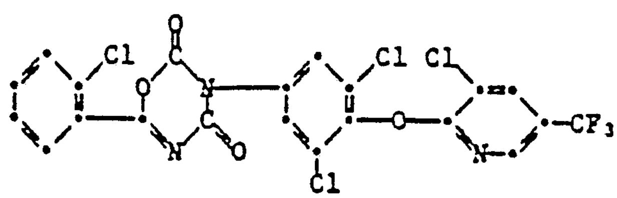

11. Produit pesticide selon la revendication 5, contenant en tant que composant actif le composé de formule

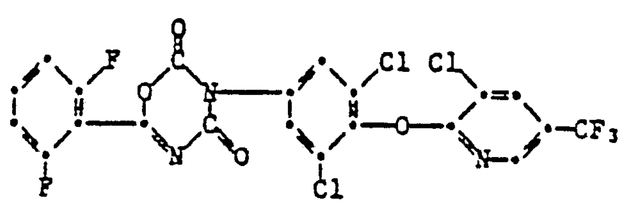

12. Produit pesticide selon la revendication 5, contenant en tant que composant actif le composé de formule

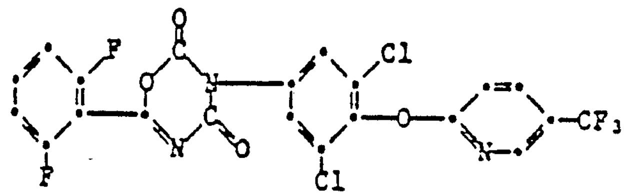

13. Procédé de préparation d'un composé de formule I

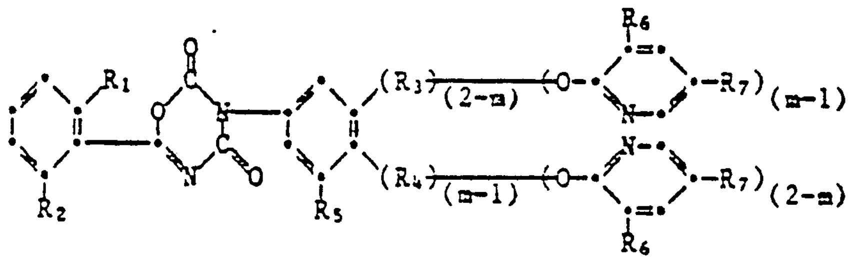

(I),

dans laquelle

$R_1$ représente un halogène, un groupe alkyle en $C_1$-$C_4$, alcoxy en $C_1$-$C_4$ ou alkylthio en $C_1$-$C_4$,
$R_2$ représente l'hydrogène, un halogène, un groupe alkyle en $C_1$-$C_4$, alcoxy en $C_1$-$C_4$ ou alkylthio en $C_1$-$C_4$,
$R_3$ et $R_4$ représentent chacun l'hydrogène, un halogène ou un groupe alkyle en $C_1$-$C_4$,
$R_5$ représente l'hydrogène, un halogène, un groupe alkyle en $C_1$-$C_4$ ou -$COOR_8$,
$R_6$ représente l'hydrogène ou un halogène,
$R_7$ représente un groupe alkyle en $C_1$-$C_4$ mono- ou polyhalogéné,
$R_8$ représente l'hydrogène ou un groupe alkyle en $C_1$-$C_4$ et
m est égal à 1 ou 2,

caractérisé en ce que l'on fait réagir de manière connue un isocyanate de benzoyle de formule II

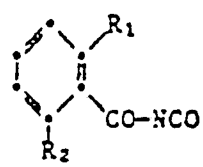

(II)

avec un isocyanate de formule III

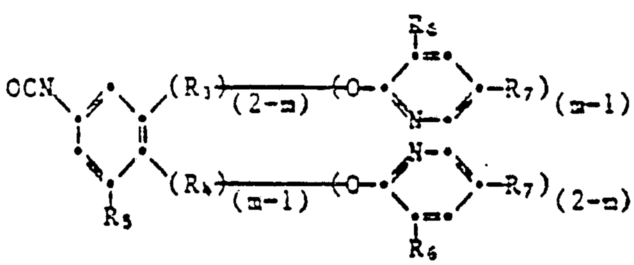

(III)

les symboles $R_1$, $R_2$, $R_3$, $R_4$, $R_5$, $R_6$, $R_7$ et m ayant dans les formules II et III les significations indiquées en référence à la formule I.

14. Procédé selon la revendication 13 pour la préparation d'un composé de formule I, caractérisé en ce que, dans la formule I,

$R_1$ représente un halogène, un groupe alkyle en $C_1$-$C_4$,
$R_2$ représente l'hydrogène, un halogène,
$R_3$ et $R_4$ représentent chacun l'hydrogène, un halogène ou un groupe alkyle en $C_1$-$C_4$,
$R_5$ représente l'hydrogène, un halogène, un groupe alkyle en $C_1$-$C_4$ ou -$COOR_8$,
$R_6$ représente l'hydrogène ou un halogène,

$R_7$ représente un groupe alkyle en $C_1$-$C_4$ mono- ou polyhalogéné,
$R_8$ représente l'hydrogène ou un groupe alkyle en $C_1$-$C_4$ et
m est égal à 1 ou 2.

15. Procédé selon la revendication 14 pour la préparation d'un composé de formule I, caractérisé en ce que, dans la formule I,

$R_1$ représente un halogène ou un groupe alkyle en $C_1$-$C_4$,
$R_2$ représente l'hydrogène, un halogène,
$R_3$ $R_4$ et $R_5$ représentent chacun l'hydrogène, un halogène ou un groupe alkyle en $C_1$-$C_4$,
$R_6$ représente l'hydrogène ou un halogène,
$R_7$ représente un groupe alkyle en $C_1$-$C_4$ mono- ou polyhalogéné,
m est égal à 1 ou 2.

16. Composé de formule I

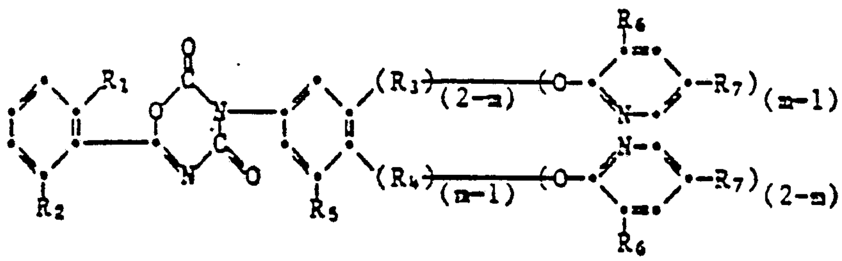

(I),

dans laquelle

$R_1$ représente un halogène, un groupe alkyle en $C_1$-$C_4$, alcoxy en $C_1$-$C_4$ ou alkylthio en $C_1$-$C_4$,
$R_2$ représente l'hydrogène, un halogène, un groupe alkyle en $C_1$-$C_4$, alcoxy en $C_1$-$C_4$ ou alkylthio en $C_1$-$C_4$,
$R_3$ et $R_4$ représentent chacun l'hydrogène, un halogène ou un groupe alkyle en $C_1$-$C_4$,
$R_5$ représente l'hydrogène, un halogène, un groupe alkyle en $C_1$-$C_4$ ou $-COOR_8$,
$R_6$ représente l'hydrogène ou un halogène,
$R_7$
représente un groupe alkyle en $C_1$-$C_4$ mono- ou polyhalogéné,
$R_8$ représente l'hydrogène ou un groupe alkyle en $C_1$-$C_4$ et
m est égal à 1 ou 2,
pour l'utilisation en tant que produit pesticide sur des animaux et des végétaux.

17. Utilisation d'un composé de formule I selon la revendication 1 dans la lutte contre les insectes et les arachnides nuisibles pour les végétaux.